# EUROPEAN PATENT SPECIFICATION

(11) **EP 3 591 039 B1**
(45) Date of publication and mention of the grant of the patent: **08.07.2026**
(21) Application number: 19772638.3
(22) Date of filing: 26.04.2019
(51) Int. Cl.: C12N 5/0793, A61K 35/30, A61P 25/16, C12N 5/00

(54) **METHOD FOR ISOLATING DOPAMINE NEURONS AND PHARMACEUTICAL COMPOSITION FOR TREATING PARKINSON'S DISEASE, CONTAINING DOPAMINE NEURONS ISOLATED USING SAME**
VERFAHREN ZUR ISOLIERUNG VON DOPAMINNEURONEN UND PHARMAZEUTISCHE ZUSAMMENSETZUNG ZUR BEHANDLUNG VON MORBUS PARKINSON UNTER VERWENDUNG DESSELBEN VERFAHRENS ISOLIERTEN DOPAMINNEURONEN
PROCÉDÉ D'ISOLEMENT DE NEURONES DE DOPAMINE ET COMPOSITION PHARMACEUTIQUE POUR LE TRAITEMENT DE LA MALADIE DE PARKINSON, CONTENANT DES NEURONES DE DOPAMINE ISOLÉS À L'AIDE DE CELUI-CI

(30) Priority: 02.05.2018 KR 20180050918; 25.04.2019 KR 20190048784
(43) Date of publication of application: 08.01.2020
(73) Proprietor: S-BIOMEDICS, Seoul 04797 (KR)
(72) Inventor: KIM, Dong-Wook, Seoul 04095 (KR); YOO, Jeong-Eun, Uijeongbu-si Gyeonggi-do 11685 (KR); LEE, Dongjin, Seoul 06077 (KR); PARK, Sanghyun, Seoul 01195 (KR); KIM, Jongwan, Seoul 02814 (KR); CHO, Myung Soo, Seoul 01488 (KR)
(74) Representative: J A Kemp LLP
(86) International application number: PCT/KR2019/005058
(87) International publication number: WO 2019/212201

(56) References cited:
- WO-A1-2012/162124
- WO-A1-2013/067362
- KR-A- 20040 079 669
- KR-B1- 100 880 948
- KR-B1- 101 331 034
- US-A1- 2005 260 591
- US-A1- 2016 256 495
- SONJA KRIKS ET AL: "'Floor plate-derived dopamine neurons from hESCs efficiently engraft in animal models of PD.'", NATURE, vol. 480, no. 7378, 6 November 2011 (2011-11-06), London, pages 547 - 551, XP055271235, ISSN: 0028-0836, DOI: 10.1038/nature10648
- GENNET, N. ET AL.: "FolR1: a novel cell surface marker for isolating midbrain dopamine neural progenitors and nascent dopamine neurons", SCIENTIFIC REPORTS, vol. 6, no. 1, 1 September 2016 (2016-09-01), pages 32488, XP055643305

## Description

### Technical Field

The present disclosure relates to a method for preparation of dopaminergic neural cells and a pharmaceutical composition comprising trophoblast glycoprotein (TPBG)-positive dopaminergic neural cells for use in treatment of Parkinson's disease.

### Background Art

Parkinson's disease (PD) is one of the most felicitous neurodegenerative disorders for cell-based therapies due to the focal degeneration of midbrain dopaminergic (mDA) neurons.

Since the early 1980s, efforts have been made to restore Parkinson's disease-related motor functions by engrafting fetal ventral mesencephalon (VM) tissue to patient's striatum.

Although the graft-induced recovery of motor functions was achieved, inconsistent outcomes were reported among research institutes or organizations, even with the production of side effects for some outcomes. Thereafter, a consensus regarding the need of improvement for cell-based treatments has been established.

Particularly, research has been continued to develop solutions to the problems indicated in previous studies, that is, to advance the grafting material cells to a level similar to that of the cells existing in the living body and avoid disadvantages of fetal tissue which includes limited availability and batch-to-batch inconsistency.

As a result, human pluripotent stem cells (hPSCs) including embryonic stem cells (ESCs) and induced pluripotent stem cells (iPSCs) have attracted attention for their abundance and ability to limitlessly proliferate *in vitro* and differentiate into a variety of neural cells.

Accordingly, techniques for differentiation into mDA neurons have been developed and advanced in recent years to a level of producing cells at high yield. However, the hPSC-derived mDA cell populations induced by recently developed differentiation techniques were found yet to be subject to the risk of heterogeneity, that is, to have mixed different kinds of cells in addition to mDA neurons. Thus, clinical application based on the current differentiation techniques is premature to consider.

Thus, identification and isolation of hPSC-derived mDA cells from diverse cell populations are critical for standardization of the cell source and successful transplantation.

The initial strategies for purely isolating (enriching) differentiated mDA cells from hPSCs were based on fluorescence-activated cell sorting (FACS) targeting multiple surface antigens. Although these early approaches enriched neuronal population expressing tyrosine hydroxylase (TH), it was unclear whether these neurons retained mDA neuronal properties.

Recent studies took much more sophisticated approaches by analyzing transcriptome of mouse fVM tissue and mouse embryonic stem cell (mESC)-derived mDA neuronal progenitors to identify specific surface markers that could distinguish and enrich mDA neurons.

While these studies suggest some feasible cell surface markers and support the feasibility of dopaminergic neuron isolation and enrichment for standardization by using the markers, it has not yet been made clear which stage of the neural cell development expresses pertinent cell surface markers that should be excavated. Because it is a common view in this field that the isolated neural cells vary in post-grafting survival rate and differentiation extent, depending on developmental stages thereof, and have an influence on post-grafting function restoration, identification of differentiation stage-specific markers is also a critical task.

Therefore, there is an urgent need for research into the differentiation stage of mDA neurons and the surface marker of the differentiation stage.

Kriks et al. (Nature 480.7378 (2012): 547-551) relates to floor plate-derived dopamine neurons from hESCs that efficiently engraft in animal models of PD.

### Summary of the Invention

The invention provides a method for preparation of dopaminergic neural cells, the method comprising: (a) contacting a cell population comprising dopaminergic neural progenitors or dopaminergic neural precursor cells with TPBG (trophoblast glycoprotein) antibodies; and (b) separating TPBG-positive dopaminergic neural cells, which bind to the TPBG antibodies.

The invention also provides a pharmaceutical composition comprising TPBG (trophoblast glycoprotein)-positive dopaminergic neural cells for use in treatment of Parkinson's disease, wherein the TPBG-positive dopaminergic neural cells are generated according to method of the invention.

The invention also provides an in vitro method for enhancing the efficacy and improving transplantation safety of dopaminergic neural cells in transplantation for Parkinson's disease, the method comprising: (a) contacting a cell population comprising dopaminergic neural progenitors or dopaminergic neural precursor cells with TPBG (trophoblast glycoprotein) antibodies; and (b) separating TPBG-positive dopaminergic neural cells, which bind to the TPBG antibodies.

### Detailed description

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

The present inventors endeavored to develop a differentiation protocol with stepwise specifications of mDA neurons and develop cell surface markers in each stage. As a result, an LMX1A-eGFP and a PITX3-mCherry reporter hESC lines were established and differentiated to isolate LMX1A⁺ mDA progenitors and PITX3⁺ mDA neurons from which an mDA neuron-related cell surface marker (TPBG) was identified.

In greater detail, the present inventors established an LMX1A-eGFP reporter hESC line which is manipulated to express green fluorescent protein (eGFP) concurrently with LMXIA, which is an mDA neural progenitor stage-specific gene, and a PITX3-mCherry reporter hESC line which is manipulated to express a red fluorescent protein (mCherry) concurrently with PITX3, which is a mature mDA neuronal stage-specific gene. Transcriptome analysis of LMX1A⁺ mDA neural precursor cells and PITX⁺ mDA neural cells revealed cell surface marker candidates specifically expressed on progenitors of mDA neural cells (neural precursor cells). Among them, TPBG was discovered as a novel cell surface marker. Cell separation targeting TPBG revealed the enrichment of mDA neural precursor cells. In addition, magnetic-activated cell sorting (MACS)-purified TPBG-positive cells at the mDA neural precursor cell stage were transplanted into 6-OHDA-lesioned Parkinson's disease (PD) rat models, with the significant recovery of motor deficits without tumor formation in the PD rats.

Therefore, the identification and use of TPBG as a new surface marker protein to isolate transplantable mDA neural precursor cells is expected to provide a safe and effective cell replacement therapy for PD.

The present disclosure relates to a method for separation of dopaminergic neural cells, a pharmaceutical composition comprising dopaminergic neural cells separated using the same method, a method for advancing the efficacy of dopaminergic neurons in cell replacement therapy for Parkinson's disease and enhancing transplantation safety, and a composition comprising TPBG (trophoblast glycoprotein)-positive dopaminergic neurons for dopaminergic neuron replacement.

Below, a detailed description will be given of the present disclosure.

An embodiment of the present disclosure pertains to a method for preparing dopaminergic neural cells, the method comprising the following steps: (a) contacting a cell population comprising dopaminergic neural progenitors or dopaminergic neural precursor cells with an TPBG (trophoblast glycoprotein) antibodies; and (b) separating TPBG-positive dopaminergic neural cells, which bind to the TPBG antibodies.

As used herein, the term "neural cells" refers to cells constituting the nervous system and is used in the same meaning as neurons, and "dopaminergic neural cells" means neural cells secreting the neurotransmitter dopamine.

The dopaminergic neural cells may be dopaminergic neural progenitors or dopaminergic neural precursor cells.

As used herein, the term "neural progenitors or neural precursor cells" means undifferentiated precursor cells that have not yet expressed a differentiated characteristic, and "progenitors", "precursors", and "precursor cell" may be used interchangeably.

The dopaminergic neural cells may be midbrain dopaminergic neural cells.

As used herein, the term "mDA neural cells" refers to dopaminergic neural cells observed in the midbrain region, for example, dopaminergic neural cells observed in the midbrain ventral region, but are not limited thereto.

Further, the mDA neural cells may be A9 region specific.

The "A9 region" is a midbrain ventrolateral region, which corresponds to the pars compacta part of the substantia nigra. Hence, the cells prepared by the preparation method of the present disclosure are midbrain cells.

The A9 region is an area in which dopaminergic neural cells are abundantly found and is related to the control of motor function. Particularly for PD patients, dopaminergic neural cells are specifically degenerated in this region. The cells prepared by the preparation method of the present disclosure may be used for preventing and/or treating PD.

The method for preparing dopaminergic neural cells of the present disclosure is described in detail, below.

### Step (a)

The "cell population" comprises dopaminergic neural progenitors or dopaminergic neural precursor cells. The dopaminergic neural progenitors may be derived from human stem cells or precursors/progenitors.

In detail, examples of the human stem cells or precursors may include embryonic stem cells, embryonic germ cells, embryonic carcinoma cells, induced pluripotent stem cells (iPSCs), adult stem cells, and fetal cells, but are not limited thereto.

The fetal cells may be derived from a fetal neural tissue and/or derivatives thereof and may be, for example, fetal ventral mesencephalic cells (fVM cells), but not limited thereto.

### Step (b)

The "TPBG" may be used in the same meaning as in Wnt-Activated Inhibitory Factor 1 or WAIF1 and is known as an antagonist of the Wnt/β-catenin signaling pathway. However, there have been no reports on the separation of dopaminergic neural cells through the expression of TPBG.

The gene has the nucleotide sequence represented by SEQ ID NO: 53. In addition, the gene may be easily available to a person skilled in the art because the nucleotide sequence is registered in the GenBank.

As used herein, the term "TPBG-positive dopaminergic neural cells" means dopaminergic neural cells bound by a TPBG antibody.

The term "TPBG antibody", as used herein, refers to an antibody that binds specifically to TPBG.

So long as it targets cells, any separation method for TPBG-positive dopaminergic neural cells can be used in this step. For example, the separation may utilize fluorescence-activated cell sorting (FACS) and/or magnetic-activated cell sorting (MACS), but is not limited thereto.

The TPBG-positive dopaminergic neural cells can alleviate symptoms of Parkinson's disease.

The TPBG-positive dopaminergic neural cells can advance the safety for cell replacement therapy.

Another embodiment of the present disclosure pertains to a pharmaceutical composition comprising TPBG (trophoblast glycoprotein)-positive dopaminergic neural cells for use in treatment of Parkinson's disease, wherein the TPBG-positive dopaminergic neural cells are generated according to the method of the invention.

The pharmaceutical composition according to the present disclosure may include a pharmaceutically acceptable carrier in addition to the effective ingredient. In this regard, the pharmaceutically acceptable carrier is typically used in preparations and may include lactose, dextrose, sucrose, sorbitol, mannitol, starch, acacia gum, calcium phosphate, alginate, gelatin, calcium silicate, microcrystalline cellulose, polyvinyl pyrrolidone, cellulose, water, syrup, methyl cellulose, methylhydroxy benzoate, propyl hydroxybenzoate, talc, magnesium stearate, and mineral oil, but is not limited thereto. In addition, ingredients such as, a lubricant, a humectant, a sweetener, a flavorant, an emulsifier, a suspending agent, a preservative, etc. may be included.

The composition of the present disclosure may be administered orally or parenterally (for example, intravenously, subcutaneously, intraperitoneally, or topically) depending on the intended method, and the dosage may vary depending on the condition and the weight of the patient, the degree of disease, the type of drug, the administration route and time, but may be appropriately selected by those skilled in the art.

The pharmaceutical composition of the present disclosure is administrated at a pharmaceutically effective dose. As used herein, the term "pharmaceutically effective dose" means an amount that is sufficient to treat the diseases at a reasonable benefit/risk ratio applicable to medical treatment or improvement, and an effective dose level may be determined according to elements including a kind of disease of the patient, the severity, age, and sex of the patient, activity of a drug, sensitivity to a drug, a time of administration, a route of administration, and an emission rate, duration of treatment, and simultaneously used drugs and other elements well-known in the medical field.

The composition of the present disclosure may be administered as an individual therapeutic agent or in combination with other therapeutic agents, may be administered sequentially or concurrently with conventional therapeutic agents, and may be administered singly or multiply. It is important to administer an amount at which the maximum effect is able to be obtained with a minimum amount without causing side effects in consideration of all of the above-described factors, and may be easily determined by those skilled in the art.

Specifically, the effective amount of the pharmaceutical composition of the present disclosure may be dependent on a patient's age, sex, condition, and body weight, an absorption rate of the active ingredient in the body, an inactivation rate, an excretion rate, a type of disease, or a drug used in combination.

The term "subject" used herein refers to a subject in need of treatment, and more specifically, a mammal such as a human, or a non-human primate, a mouse, a rat, a dog, a cat, a horse, and a cow.

Another embodiment of the present disclosure pertains to an in vitro method for enhancing the efficacy and improving transplantation safety of dopaminergic neural cells in transplantation for Parkinson's disease, the method comprising the following steps:
(a) contacting a cell population comprising dopaminergic neural progenitors or dopaminergic neural precursor cells with a TPBG (trophoblast glycoprotein)-antibodies; and
(b) separating TPBG-positive dopaminergic neural cells, which bind to the TPBG antibodies.

In order to avoid undue redundancy leading to the complexity of this specification by the method for advancing the efficacy of dopaminergic neurons and enhancing transplantation safety because it uses the same elements as described in the preparation method for dopaminergic neural cells, the common description between them are omitted.

The TPBG-positive dopaminergic neural cells can be cultured by the preparation method for dopaminergic neural cells, and the TPBG-positive dopaminergic neural cells cultured by the method may be enhanced in cell efficacy and improved in transplantation safety.

For transplantation of the dopaminergic neural cells, an appropriate injection site (e.g., the putamen or caudate nucleus, or the striatum including both of them in the brain) is selected and then, a well-known modality (for example, stereotactic system, etc.) may be taken.

The composition of the present disclosure may be used in treating Parkinson's disease.

The composition of the present disclosure may comprise dopaminergic neural cells as transplanted cells, alone or in combination with a biocompatible and/or biodegradable stabilizer. The stabilizer functions to stably disperse the dopaminergic neural cells and is a bio-derived material that does not cause any side effect after transplantation. Alternatively, the stabilizer should be biodegradable. As used herein, the term "biodegradable" refers to having the property of being slowly degraded in and absorbed into the body, but does not impose special meaning on a degradation speed.

Examples of the stabilizer include hyaluronic acid, collagen, thrombin, elastin, chondroitin sulfate, albumin, and a mixture thereof. Particularly, hyaluronic acid, collagen, thrombin, elastin, chondroitin sulfate, and albumin are bio-derived materials that have biodegradability, e.g., are naturally degraded *in vivo.* So long as it meets the requirement for being biodegradable and providing viscosity in a medium, even a synthetic compound may be used in the present disclosure. Hence, the stabilizer is not limited only to bio-derived materials.

When formulated together with the stabilizer, dopaminergic neural cells do not float or settle, but can exist in an evenly dispersed form.

In order to avoid undue redundancy leading to the complexity of this specification by the composition comprising TPBG (trophoblast glycoprotein)-positive dopaminergic neurons for dopaminergic neuron replacement because it uses the same elements as described in the preparation method, the common description between them are omitted.

### Advantageous Effects

The present disclosure addresses a method for preparation of dopaminergic neural cells and a pharmaceutical composition comprising the dopaminergic neural cells generated by the method for use in treatment of Parkinson's disease, wherein the method comprises a step of separating TPBG-positive dopaminergic neural cells, whereby the dopaminergic neural cells separated according to the method of the present disclosure are enhanced in efficacy for transplantation and have advanced transplantation safety and thus can find useful applications in transplantation for Parkinson's disease.

### Brief Description of the Drawings

FIG. 1 is a schematic diagram of a method for preparation of dopaminergic neural cells.
FIG. 2 is a view schematically illustrating a method for construction of LMX1A-eGFP reporter hESC lines.
FIG. 3 is a view schematically illustrating a method for construction of PITX3-mCherry reporter hESC lines according to a Preparation Example of the present disclosure
FIG. 4 shows views identifying a procedure of mDA neural precursor differentiation of the LMX1A-eGFP reporter hESC lines constructed according to a Preparation Example of the present disclosure.
FIG. 5 shows views identifying a procedure of mDA neuronal cell (neuron) differentiation of the PITX3-mCherry reporter hESC lines constructed according to a Preparation Example of the present disclosure.
FIGS. 6a and 6b are views characterizing LMX1A-expressing mDA neural precursor cells differentiated according to an Example of the present disclosure.
FIGS. 7a and 7b are views characterizing LMX1A-expressing mDA neural precursor cells (II) differentiated according to an Example of the present disclosure.
FIGS. 8a and 8b are views characterizing post-terminal differentiation LMX1A-expressing cells differentiated according to an Example of the present disclosure.
FIGS. 9a to 9c are views characterizing PITX3-expressing mDA neuronal cells (neurons) differentiated according to an Example of the present disclosure.
FIG. 10 shows views characterizing PITX3-expressing mDA neuronal cells (neurons) differentiated according to an Example of the present disclosure.
FIG. 11 shows views comparing *in vitro* cell viability between LMX1A-expressing mDA neural precursor cells and PITX3-expressing mDA neuronal cells (neurons), both differentiated according to an Example of the present disclosure.
FIG. 12 shows views accounting for results of the transcriptome analysis performed with regard to LMX1A-expressing mDA neural precursor cells and PITX3-expressing mDA neuronal cells (neurons), both differentiated according to an Example of the present disclosure.
FIG. 13 is a schematic diagram illustrating a procedure of identifying candidates of mDA markers.
FIGS. 14 and 15 show evaluation results of target validation for identifying candidates of mDA markers.
FIGS. 16 and 17 are views accounting for results of MACS targeting candidates (CORIN, TPBG, CD47, ALCAM) of mDA markers.
FIG. 18 is a view accounting for behavioral recovery in PD animal models after transplantation of TPBG-positive cells derived from hESC according to an Example of the present disclosure.
FIG. 19 shows views characterizing grafts in PD-animal models after transplantation of TPBG-positive cells derived from hESC according to an Example of the present disclosure.
FIG. 20 shows views identifying cell proliferative potentials of unsorted cell grafts compared with TPBG-positive cell grafts in PD-animal models after transplantation of TPBG-positive cells derived from hESC according to an Example of the present disclosure.
FIG. 21 is a view characterizing TPBG-positive cells separated from human fVM cells according to an Example of the present disclosure.
FIG. 22 shows views characterizing TPBG-positive cells derived from human iPSC according to an Example of the present disclosure.

### Mode for the Disclosure

### Human Embryonic Stem Cell (hESC) Culture

Undifferentiated hESCs (H9; WiCell Inc., USA) were cultured in Dulbecco's modified Eagle's medium (DMEM)/F12 (Gibco-Thermo Fisher Scientific) supplemented with 20% knockout-serum replacement (KSR) (Invitrogen, USA) , 1x nonessential amino acid (Gibco-Thermo Fisher Scientific, USA), 0.1 mM β-mercaptoethanol (Sigma-Aldrich), and 4 ng/ml of basic fibroblast growth factor (bFGF) (R&D System, USA) on the layer of mitomycin-C (Sigma-Aldrich, USA) treated mouse STO fibroblasts (ATCC, USA). The use of H9 cells is for reference only and not according to the claimed invention.

### Genotyping of Clonal Cell

Genomic DNA was extracted using DNeasy Blood & Tissue kit (QIAGEN, Germany) according to the manufacturer's instruction. Genomic DNA PCR was performed with EmeraldAmp^{®} GT PCR Master Mix (TAKARA Bio Inc., Japan) in the GeneAmp PCR System 2720 (Applied Biosystems-Thermo Fisher Scientific).

### FACS

FACS was performed using BD FACSAria III cell sorter and FACSDiva software (BD Bioscience). Using a 488-nm laser, an eGFP-positive fraction was determined depending on fluorescence intensity. Using a 561-nm laser, an mCherrypositive fraction was determined depending on fluorescence intensity.

### MACS

In order to block antibodies from binding nonspecifically thereto, cells were incubated in 1% FBS-PBS solution (4°C, 30 min) and then with primary antibodies (see Table 1, below) for 30 min at 4°C.

**TABLE 1**

| Protein | Species | Company | Cat. no. | Dilution |
|---|---|---|---|---|
| OCT4 | Rabbit | Santa Cruz | sc-9081 | 1:200 |
| SOX2 | Rabbit | Millipore | AB5603 | 1:200 |
| NANOG (human) | Goat | R&D Systems | AF1997 | 1: 50 |
| SSEA4 | Mouse | Millipore | MAB4304 | 1:200 |
| TRA-1-81 | Mouse | Millipore | MAB4381 | 1:100 |
| TRA-1-60 | Mouse | Millipore | MAB4360 | 1:100 |
| NESTIN (human) | Rabbit | Millipore | ABD69 | 1:1,000 |
| SOX1 | Goat | R&D Systems | AF3369 | 1:100 |
| SMAα | Mouse | SIGMA | A5228 | 1:100 |
| BRACHYURY | Goat | R&D Systems | AF2085 | 1:100 |
| EN1 | Mouse | Dev. Stud. Hybridoma Bank | 4G11 | 1:50 |
| FOXA2 (HNF3β) | Rabbit | Abcam | AB108422 | 1:300 |
| FOXA2 (HNF3β) | Goat | Santa Cruz | sc-6554 | 1:100 |
| LMX1A | Goat | Santa Cruz | sc-54273 | 1:100 |
| eGFP | Goat | Rockland | 600-101-215 | 1:1, 000 |
| eGFP | Mouse | Rockland | 600-301-215 | 1:1,000 |
| PITX3 | Rabbit | NOVUS | NBP1-92274 | 1:500 |
| mCherry | Rabbit | Rockland | 600-401-P16S | 1:1, 000 |
| mCherry | Rat | Thermo | M11217 | 1:1,000 |
| KI67 | Rabbit | Vision Biosystem | NCL-K67P | 1:1, 000 |
| TUBB3 | Mouse | Covance (BioLegend) | MMS-435P (801201) | 1:1, 000 |
| TH | Rabbit | Pel-freez | P40101-0 | 1:1, 000 |
| TH | Mouse | Sigma | T1299 | 1:10, 000 |
| NURR1 | Rabbit | Santa Cruz | sc-990 | 1:1,000 |
| MAP2 | Rabbit | Millipore | AB5622 | 1:1, 000 |
| AADC | Rabbit | Chemicon | AB1569 | 1:500 |
| VMAT2 | Rabbit | Abcam | AB81855 | 1:1, 500 |
| DAT | Rabbit | Pel-freez | P40501-0 | 1:500 |
| KCNJ6 | Rabbit | Almone Labs | APC-006 | 1:500 |
| CALB | Rabbit | Millipore | AB1778 | 1:1,000 |
| NCAM (human) | Mouse | Santa Cruz | sc-106 | 1:100 |
| PCNA | Rabbit | Abcam | ab18197 | 1:700 |
| PH3 | Rabbit | Millipore | 06-570 | 1:500 |
| NeuN | Mouse | Chemicon | MAB377 | 1:100 |
| NeuroD | Mouse | Abcam | AB60704 | 1:500 |
| ALCAM | Mouse | R&D Systems | MAB561 | 2.5µg/10⁶ cells |
| TPBG | Mouse | R&D Systems | MAB49751 | 2.5 µg /10⁶ cells |
| CORIN | Mouse | R&D Systems | MAB2209 | 2.5 µg /10⁶ cells |
| CD47 | Mouse | Santa Cruz | sc-12730 | 1.0 µg /10⁶ cells |

After washing, the primary antibody-labeled cells were incubated with 20 µL of microbeads (Miltenyi Biotec) per 1x10⁷ cells. After washing, the cell suspension was loaded on the separation column (LS column) (Miltenyi Biotec) that was attached to a magnetic stand. Negatively labeled cells which were passed-through during column washing were collected in a separate tube, and positively labeled cells that remained in the column were eluted to another tube, together with the culture medium after removing the column from the magnetic stand.

### Immunocytochemistry

First, cells were fixed in 4% paraformaldehyde-PBS solution.

For smooth permeabilization of antibody into cytosols, then, cells were treated with 0.1% Triton X-100-PBS solution, blocked with 2% bovine serum albumin (BSA) (Bovogen, Australia)-PBS solution for 1 hr at room temperature, and incubated overnight at 4°C with primary antibodies (see Table 1 above). Appropriate fluorescence-tagged secondary antibodies (Molecular Probes-Thermo Fisher Scientific and Vector Laboratories, USA) were used for visualization of the primary antibody-labeled protein.

Finally, in order to identify cell nuclei, cells were mounted in 4',6-diamidino-2-phenylindole mounting medium (Vector Laboratories), and images were obtained using an Olympus IX71 microscope equipped with a DP71 digital camera, Olympus FSX100 system (Olympus Corp., Japan) or LSM710 confocal microscope (Carl Zeiss, Germany).

### Flow cytometry

Cells were dissociated into single cells using Accutase (Merck Millipore, Germany) and fixed with 4% paraformaldehyde-PBS solution. For detecting intracellular markers, cells were permeabilized with 1X Perm/Wash buffer (BD Biosciences) and incubated with the appropriate antibodies for 1 hr in 2% BSA-PBS solution. Appropriate fluorescence-tagged secondary antibodies were used. Flow cytometry was performed using LSRII (BD Biosciences) and analyzed using FlowJo software.

### Gene expression analysis

Total RNA was isolated using the Easy-Spin^{®} Total RNA Extraction kit (iNtRON Biotechnology, South Korea). cDNA was synthesized from 1 µg of total RNA using the PrimeScript^{™} RT Master Mix (TAKARA Bio, Inc.). mRNA levels were quantified by real-time RT-PCR assays using the SYBR^{®} Premix Ex Taq^{™} (TAKARA Bio, Inc.) and CFX96 Real-Time System (Bio-Rad, USA). Ct values for each targeted gene were normalized according to those of GAPDH, and the normalized expression levels of the targeted genes were compared between the sorted/unsorted samples and control samples based on the comparative Ct method. Data are expressed as the mean relative expression ± standard error of the mean (SEM) from three independent experiments. The sequences of the primers used for gene expression analysis are listed in Table 2, below.

**TABLE 2**

| Symbol | Gene name | Sequence(5' to 3') | SEQ ID No. |
|---|---|---|---|
| GAPDH | Glyceraldehyde-3-Phosphate Dehydrogenase | F: CAA TGA CCC CTT CAT TGA CC | SEQ ID No.1 |
| | | R: TTG ATT TTG GAG GGA TCT CG | SEQ ID No.2 |
| OCT4 | POU class 5 homeobox 1 | F: CCT CAC TTC ACT GCA CTG TA | SEQ ID No.3 |
| | | R :CAG GTT TTC TTT CCC TAG CT | SEQ ID No. 4 |
| SOX2 | SRY-box2 | F: TTC ACA TGT CCC AGC ACT ACC AGA | SEQ ID No.5 |
| | | R: TCA CAT GTG TGA GAG GGG CAG TGT GC | SEQ ID No. 6 |
| NANOG | Nanog homeobox | F: TGA ACC TCA GCT ACA AAC AG | SEQ ID No. 7 |
| | | R: TGG TGG TAG GAA GAG TAA AG | SEQ ID No. 8 |
| TET1 | TET methylcytosine dioxygenase 1 | F: CTG CAG CTG TCT TGA TCG AGT TAT | SEQ ID No. 9 |
| | | R: CCT TCT TTA CCG GTG TAC ACT ACT | SEQ ID No. 10 |
| REX1 | ZFP42 zinc finger protein | F: TCA CAG TCC AGC AGG TGT TTG | SEQ ID No. 11 |
| | | R: TCT TGT CTT TGC CCG TTT CT | SEQ ID No. 12 |
| EN1 | Engrailed 1 | F: CGT GGC TTA CTC CCC ATT TA | SEQ ID No. 13 |
| | | R: TCT CGC TGT CTC TCC CTC TC | SEQ ID No. 14 |
| FOXA2 | Forkhead box A2 (HNF-3β) | F: CCG TTC TCC ATC AAC AAC CT | SEQ ID No.15 |
| | | R: GGG GTA GTG CAT CAC CTG TT | SEQ ID No.16 |
| LMX1A | LIM homeobox transcription factor 1a | F: CGC ATC GTT TCT TCT CCT CT | SEQ ID No. 17 |
| | | R: CAG ACA GAC TTG GGG CTC AC | SEQ ID No. 18 |
| eGFP | Enhanced green fluorescent protein | F: CAT CAA GGT GAA CTT CAA GAT CCG CCA CAA C | SEQ ID No.19 |
| | | R: CTT GTA CAG CTC GTC CAT GCC GAG AGT GAT C | SEQ ID No.20 |
| PITX3 | Paired like homeodomain 3 | F: GCC AAC CTT AGT CCG TG | SEQ ID No.21 |
| | | R: GCA AGC CAG TCA AAA TG | SEQ ID No.22 |
| mCherry | | F: ACT ACG ACG CTG AGG TCA AG | SEQ ID No.23 |
| | | R: GTG TAG TCC TCG TTG TGG GA | SEQ ID No.24 |
| OTX2 | Orthodenticle homeobox 2 | F: GGA AGC ACT GTT TGC CAA GAC C | SEQ ID No.25 |
| | | R: CTG TTG TTG GCG GCA CTT AGC T | SEQ ID No.26 |
| FOXA1 | Forkhead box A1 | F: GGG CAG GGT GGC TCC AGG AT | SEQ ID No.27 |
| | | R: TGC TGA CCG GGA CGG AGG AG | SEQ ID No.28 |
| SIM1 | Single-minded homolog 1 | F: AAA GGG GGC CAA ATC CCG GC | SEQ ID No.29 |
| | | R: TCC GCC CCA CTG GCT GTC AT | SEQ ID No.30 |
| LHX1 | LIM homeobox 1 | F: AGG TGA AAC ACT TTG CTC CG | SEQ ID No.31 |
| | | R: CTC CAG GGA AGG CAA ACT CT | SEQ ID No.32 |
| LMX1B | LIM homeobox transcription factor 1b | F: CTT AAC CAG CCT CAG CGA CT | SEQ ID No.33 |
| | | R: TCA GGA GGC GAA GTA GGA AC | SEQ ID No.34 |
| NKX2.2 | NK2 homeobox 2 | F: CCT TCT ACG ACA GCA GCG ACA A | SEQ ID No.35 |
| | | R: ACT TGG AGC TTG AGT CCT GAG G | SEQ ID No.36 |
| NKX6.1 | NK6 homeobox 1 | F: CGA GTC CTG CTT CTT CTT GG | SEQ ID No.37 |
| | | R: GGG GAT GAC AGA GAG TCA GG | SEQ ID No.38 |
| NURR1 | Nuclear receptor subfamily 4 group A member 2 | F: AAA CTG CCC AGT GGA CAA GCG T | SEQ ID No.39 |
| | | R: GCT CTT CGG TTT CGA GGG CAA A | SEQ ID No.40 |
| TH | Tyrosine hydroxylase | F: GCT GGA CAA GTG TCA TCA CCT G | SEQ ID No.41 |
| | | R: CCT GTA CTG GAA GGC GAT CTC A | SEQ ID No.42 |
| DAT | Dopamine transporter | F: CCT CAA CGA CAC TTT TGG GAC C | SEQ ID No.43 |
| | | R: AGT AGA GCA GCA CGA TGA CCA G | SEQ ID No.44 |
| VMAT2 | Solute carrier family 18 member A2(vesicular monoamine transporter 2) | F: GCT ATG CCT TCC TGC TGA TTG C | SEQ ID No.45 |
| | | R: CCA AGG CGA TTC CCA TGA CGT T | SEQ ID No.46 |
| HTR2B | 5-hydroxytryptamine receptor 2B | F: GCT GGT TGG ATT GTT TGT GAT GC | SEQ ID No.47 |
| | | R: CCA CTG AAA TGG CAC AGA GAT GC | SEQ ID No.48 |
| NeuN | RNA binding fox-1 homolog 3 | F: TAC GCA GCC TAC AGA TAC GCT C | SEQ ID No.49 |
| | | R: TGG TTC CAA TGC TGT AGG TCG C | SEQ ID No.50 |
| MAP2 | Microtubule associated protein 2 | F: AGG CTG TAG CAG TCC TGA AAG G | SEQ ID No.51 |
| | | R: CTT CCT CCA CTG TGA CAG TCT G | SEQ ID No.52 |

### Microarray: Transcriptome profiling

Ten µg of total RNA from each sample was processed and analyzed by Macrogen, Inc. (Korea), and the samples were hybridized to the Affymetrix Human U133 Plus 2.0 array.

The present disclosure will become more fully understood from the following Examples. The Examples are given by way of illustration only, and thus are not limitative of the present disclosure.

### EXAMPLE: mDA Neural Cell Differentiation Protocol

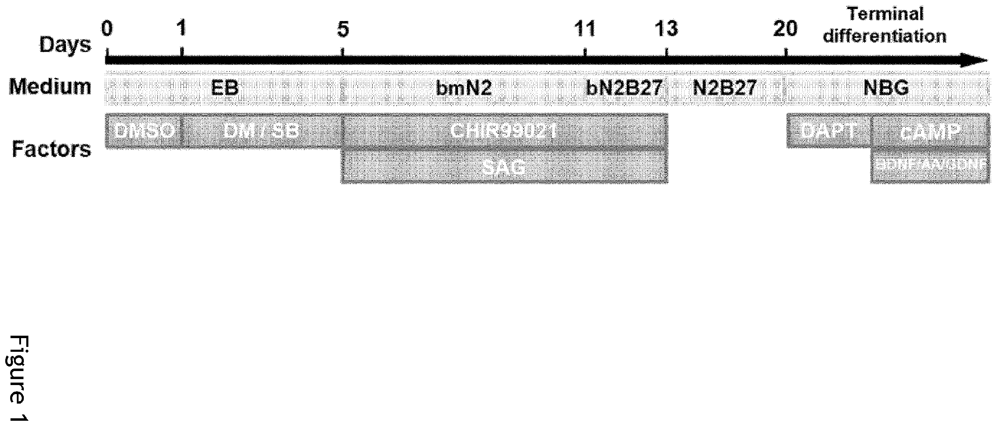

A concrete protocol is depicted in FIG. 1.

hESCs cultured in the form of colonies were detached with 2 mg/ml of type IV collagenase (Worthington Biochemical Corp., USA) for 30 min and embryoid bodies were induced to form in bFGF-free hES culture medium (EB medium) including 1.5% dimethyl sulfoxide (DMSO; Calbiochem-Merck Millipore) for the first 24hrs, followed by treating the embryoid bodies with 5 µM dorsomorphin (DM) (Calbiochem-Merck Millipore) and 5 µM SB431542 (SB) (Sigma-Aldrich) for four days.

On day five (d5), EBs were attached to Matrigel-coated culture dishes in DMEM/F12 1X N2 supplemented media containing 20 ng/mL bFGF and 20 µg/ml human insulin solution (Sigma-Aldrich) (bmN2 medium) and treated with patterning factors (1 µM CHIR99021 (Miltenyi Biotec) and 0.5 µM SAG (Calbiochem-Merck Millipore)) for another 6 days.

On day 11 (d11), the emerged rosette structures formed within the EB colonies were mechanically isolated using pulled glass pipettes and the isolated neural rosette clumps were crushed by pipetting and replated in Matrigel-coated dishes. The replated cells were then expanded and specified for an additional 2 days in DMEM/F12 1X N2 and 1X B27 media with 20 ng/mL bFGF (bN2B27 medium) supplemented with 1 µM CHIR99021 and 0.5 µM SAG to induce differentiation into mDA neural cells.

On day 13 (d13) of differentiation, the mDA neural precursor clusters were dissociated to single cells by using Accutase and replated onto Matrigel-coated plates at a density of 3.12x10⁵ cells/cm² in a bFGF-free N2B27 medium. mDA precursors were expanded in N2B27 medium for additional 7 days at 90% confluence.

On day 20 (d20), midbrain/ventral mDA neural precursors were cultured in 1X N2, 0.5X B27 and 0.5X G21 supplement (Gemini Bio- Products, USA) (NBG medium) with 1 µM of DAPT (Sigma-Aldrich) for the first 7 days. After that, the cells were cultured in NBG medium with 10 ng/ml of brain-derived neurotrophic factor (BDNF) (ProSpec-Tany TechnoGene, Israel), 10 ng/ml of glial cell line-derived neurotrophic factor (GDNF) (ProSpec-Tany TechnoGene), 200 µM of ascorbic acid (AA), and 1 µM of dibutyryl cyclic-AMP (db-cAMP) (Sigma-Aldrich) for terminal differentiation.

### PREPARATION EXAMPLE 1: Construction of LMX1A-eGFP Reporter Cell Line

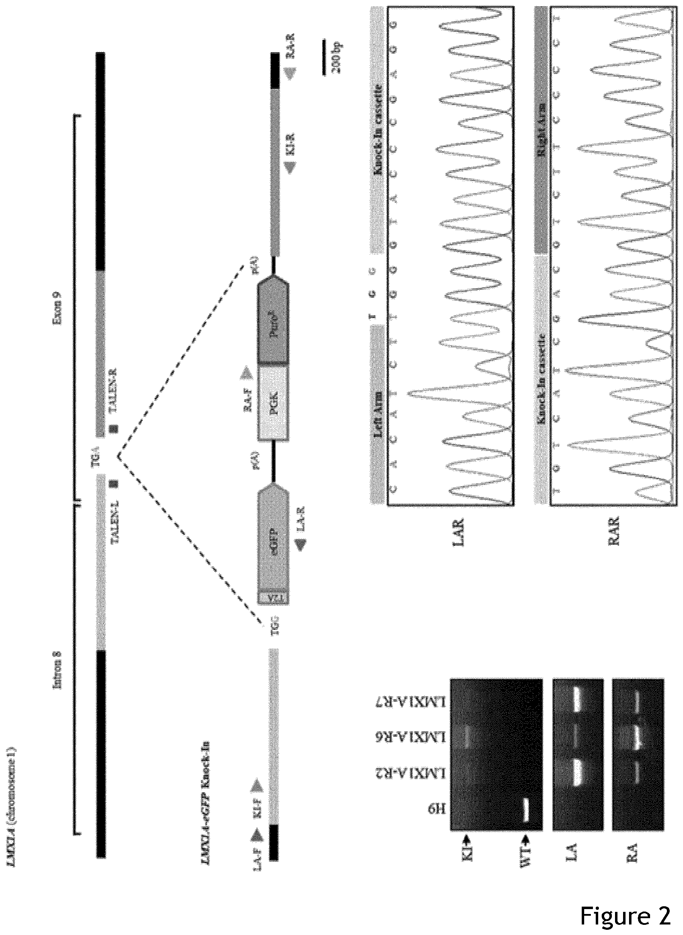

A concrete protocol is given in FIG. 2.

### 1-1. Design of programmable nucleases and donor DNA plasmids

TALEN-encoding plasmids were purchased from ToolGen, Inc. (Korea).

TALEN sites were designed to cause double-strand breaks (DSBs) near the stop codon, TGA, in exon 9 of *LMX1A* gene ( 5' - TCC ATG CAG AAT TCT TAC TT - 3' (left), 5' - TCA CAG AAC TCT AGG GGA AG - 3' (right)). Potential off-target sites were searched using Cas-OFFinder (www.rgenome.net/).

The donor DNA plasmids were constructed using pUC19 as the plasmid backbone in DH5α as follows: 5' homology arm-endogenous *LMX1A* genomic fragment (left arm)-T2A-eGFP-bGH poly(A)-PGK promoter driven puromycin resistance cassette-bGH poly(A)- 3' homology arm (right arm).

### 1-2. Generation of LMX1A-eGFP reporter line

hESC colonies on inactivated STO were transferred on plates coated with hESC-qualified Matrigel (BD Biosciences, Bedford, Massachusetts, USA) in StemMACS^{™} iPS-Brew XF complete medium (Miltenyi Biotec, Germany). Thereafter, cells were passaged when they were 80~90% confluent (split ratio, 1:5). Cells were dissociated into single cells using Accutase and transferred on Matrigel-coated plates with ROCK inhibitor (10 µM, Y-27632) (Calbiochem-Merck Millipore) included in the medium for the first 24hrs after plating and continued with daily medium changes. Only hESCs that had undergone less than 10 enzymatic passages were used in the experiments.

hESCs were harvested using Accutase to create single cell suspensions. These cells were then resuspended gently with R buffer from the Neon transfection kit (100 µl kit; Invitrogen) at a final density of 1.0x10⁷ cells/ml. 120 µl of resuspended cells were mixed with a pair of TALEN-encoding plasmids of Preparation Example 1-1 (6 µg of each plasmid) and donor LMX1A DNA plasmid and pulsed with a voltage of 850 mV for 30ms for electroporation (Neon transfection system; Invitrogen).

Cells were subsequently plated into two or three 35-mm dishes preseeded with STO feeders in hESC medium supplemented with ROCK inhibitor for the first 48 hours. The medium was changed with a fresh one after 2 days, and then the medium was changed every day.

Five days after electroporation, cells were treated 0.5 µg/ml puromycin (Sigma- Aldrich) in hESC medium. At 10-14 days after electroporation, individual colonies resistant to puromycin were picked as reporter cell line candidates and expanded by passages

Finally, genotyping of clonal cells allowed for the selection of LMX1A-eGFP reporter lines.

### PREPARATION EXAMPLE 2: Generation of PITX3-mCherry Reporter Line

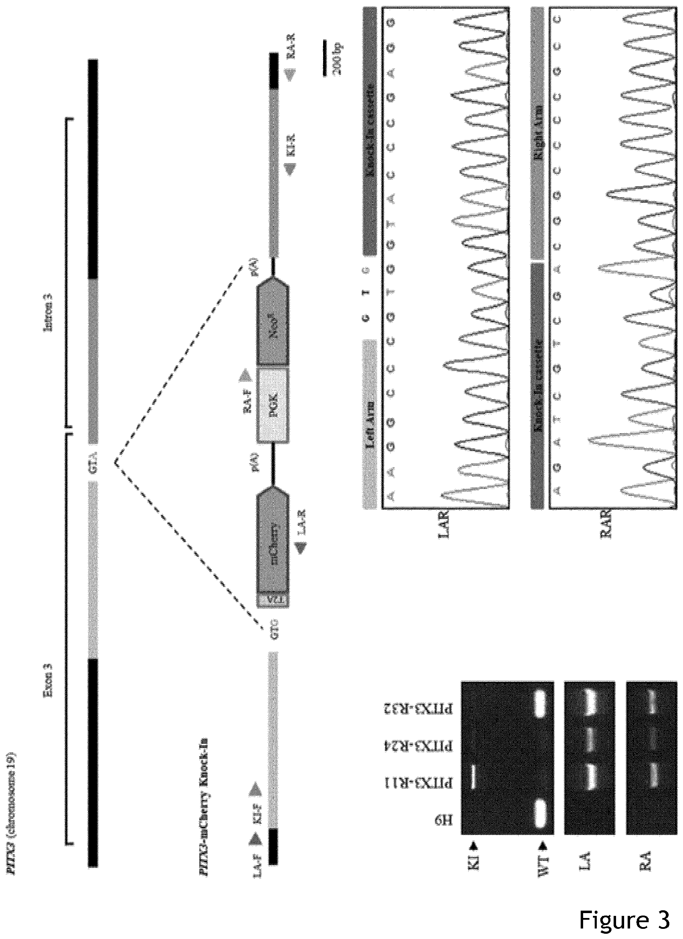

A concrete protocol is depicted in FIG. 3.

### 2-1. Design of nuclease and donor DNA plasmid

Cas9- and sgRNA (CRISPR/Cas9)-encoding plasmids were purchased from ToolGen. Inc.

The sequence for making sgRNA for mediating *PITX3* targeting was located so that it spanned across the stop codon TGA(5'-TAC GGG CGG GGC CGC TCA TAC GG-3' (PAM is underlined)) to cause double strand breaks (DSBs) near the stop codon TGA. Potential off-target sites were searched using Cas-OFFinder (www.rgenome.net/)

The donor DNA plasmids were constructed using pUC19 as the plasmid backbone in DH5α as follows: 5' homology arm-endogenous *PITX3* genomic fragment(left arm)-T2A-mCherry-bGH poly(A)-PGK promoter driven neomycin resistance cassette-bGH poly(A)-3' homology arm(right arm).

### 2-2. Generation of PITX3-mCherry reporter line

A PITX3 reporter line was generated in the same manner as in Preparation Example 1-2, with the exception that a Cas9- and sgRNA-encoding plasmid, a PITX3 donor DNA plasmid, and 100 µg/mL G418 (Calbiochem-Merck Millipore) were used instead of the TALEN-encoding plasmid, the LMX1A donor DNA plasmid, and 0.5 µg/mL puromycin, respectively.

### EXPERIMENTAL EXAMPLE 1: Identification at Progenitor Stage: Identification of LMX1A-eGFP reporter line

The LMX1A-eGFP reporter line generated in Preparation Example 1 was differentiated according to the differentiation protocol of the Example, followed by analyzing the differentiation (Immunocytochemistry and Cytometry).

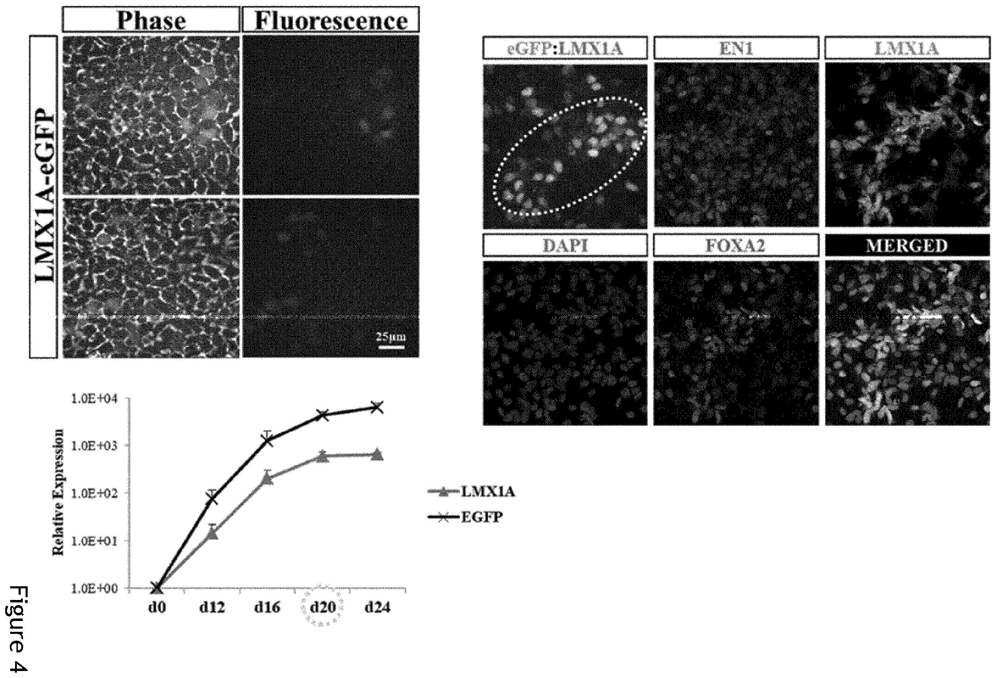

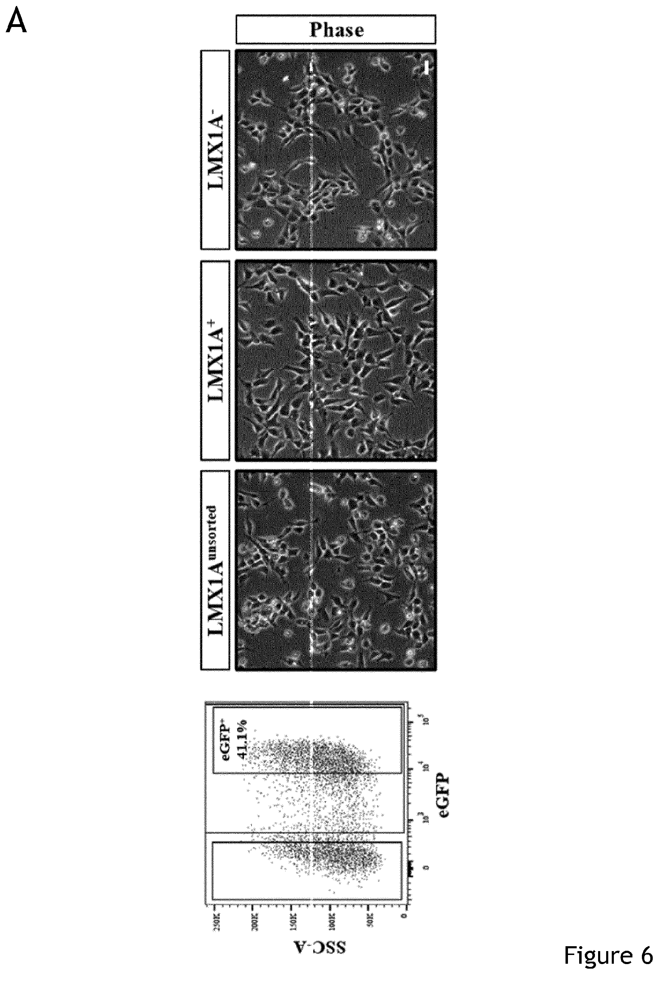

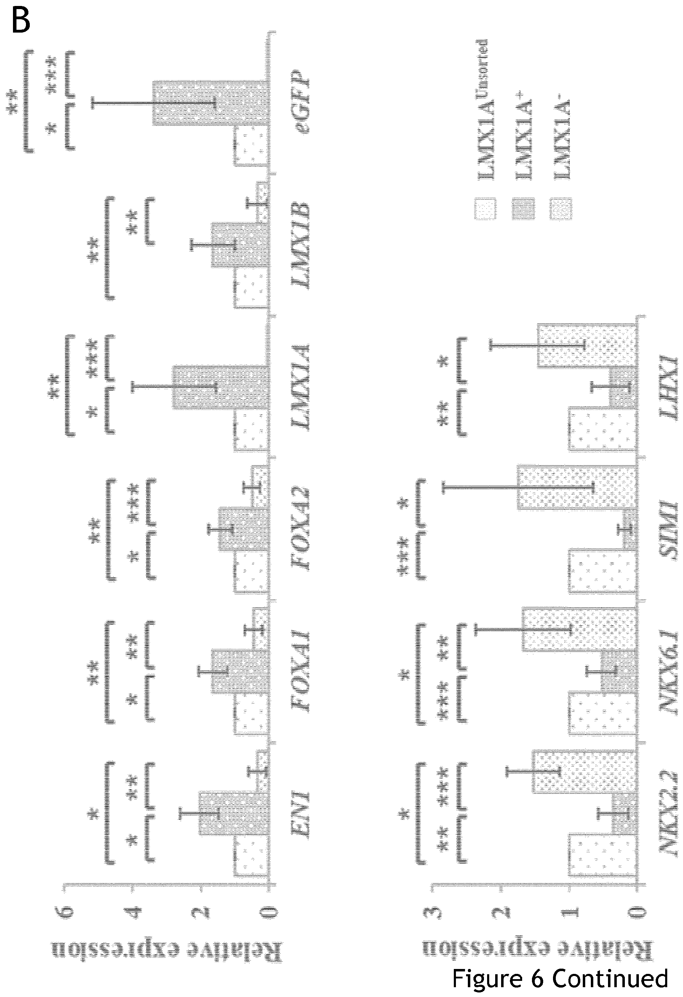

As can be seen in FIG. 4, eGFP expression was observed, together with the midbrain regional marker EN1, the midbrain floor plate regional marker FOXA2, and the dopamine lineage marker LMX1A, throughout the progenitor differentiation. Particularly, by 20 days of differentiation (d20), ~41.1% of the cell population appeared to be eGFP-positive eGFP⁺) and the eGFP⁺ cells co-expressed EN1 and FOXA2 (Figure 4 and Figure 6). Progenitors positive for all three makers (EN1, FOXA2, and LMX1A) were also detected (~46.6% EN1⁺ eGFP⁺, ~49.2% FOXA2⁺eGFP⁺ (see FIG. 3).

These data indicated that eGFP-expressing cells expressed LMX1A (construction of LMX1A reporter lines) and hESCs were directed to differentiate into mDA neural progenitors with floor plate (FOXA2) and midbrain (EN1) characteristics

### EXPERIMENTAL EXAMPLE 2: Identification at Neuronal Stage: Identification of PITX3-mCherry reporter

The PITX3-mCherry reporter line generated in Preparation Example 2 was differentiated according to the differentiation protocol of the Example, followed by analyzing the differentiation (Immunocytochemistry and Cytometry) .

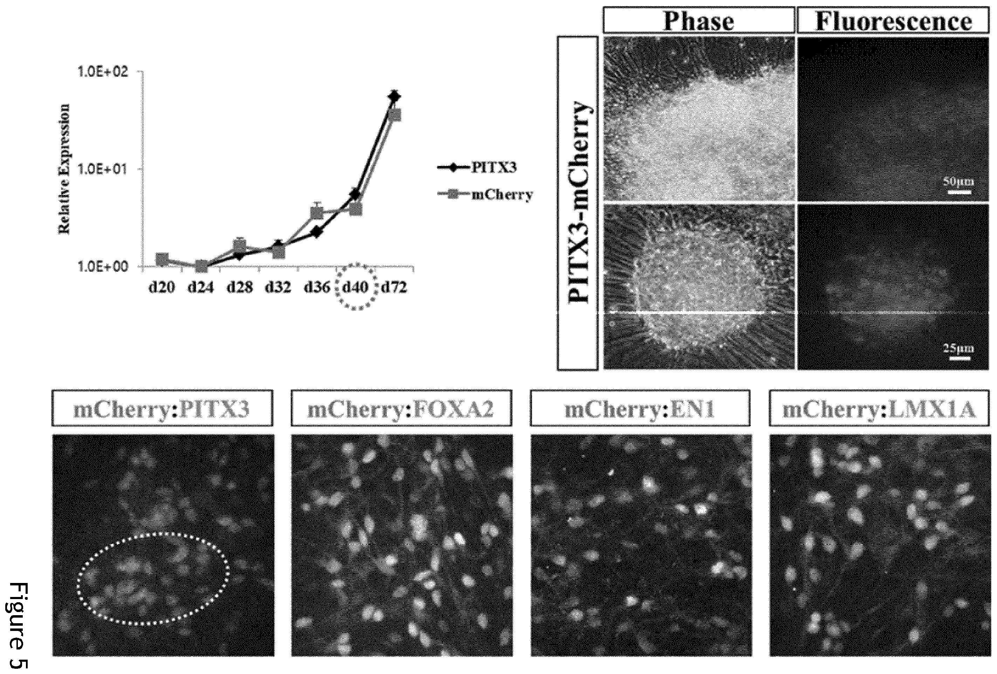

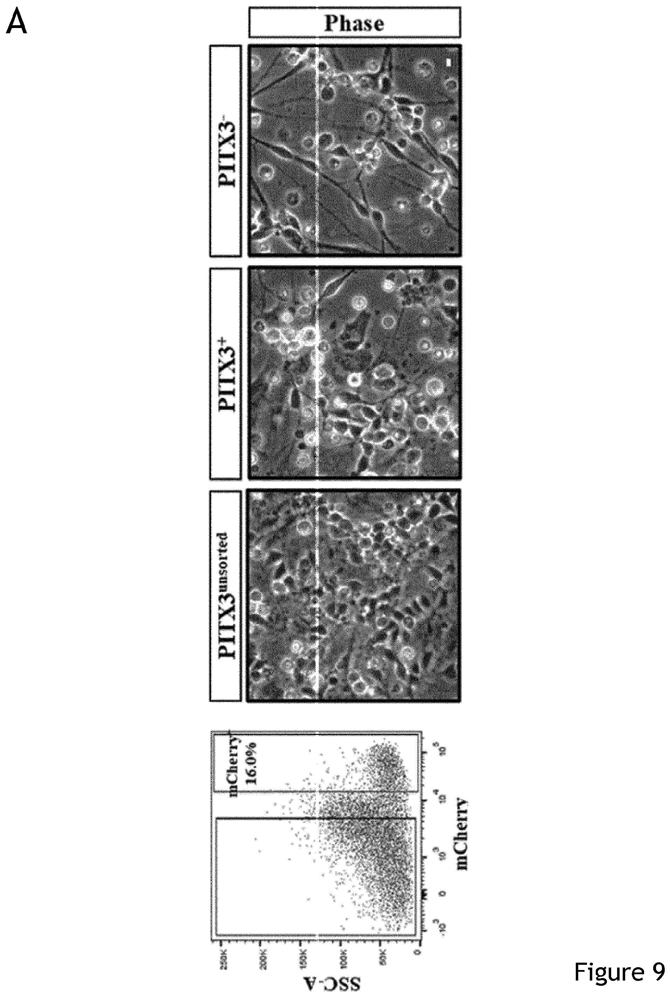

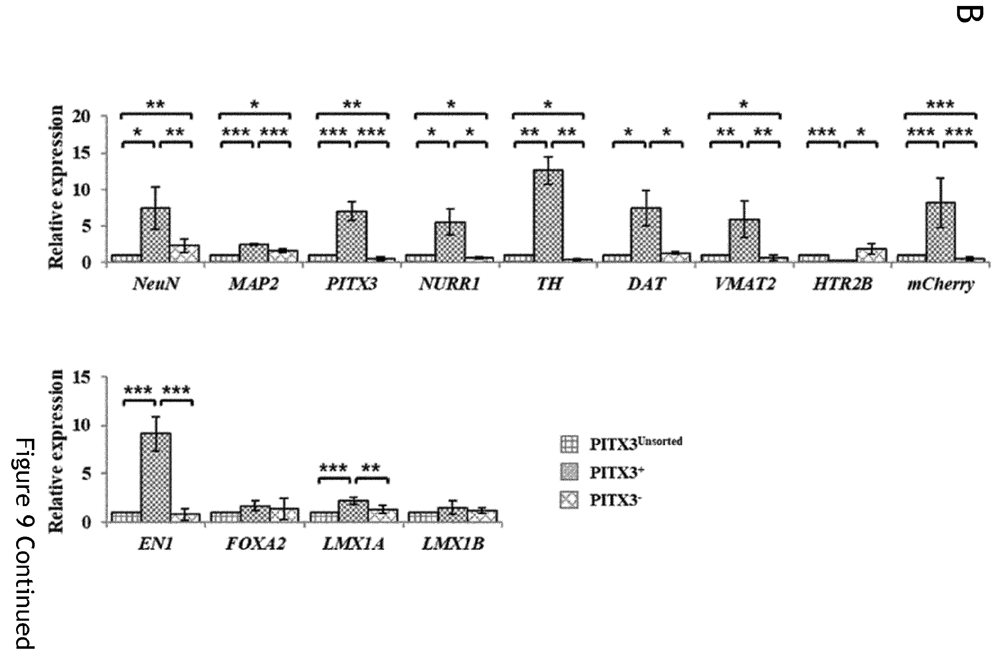

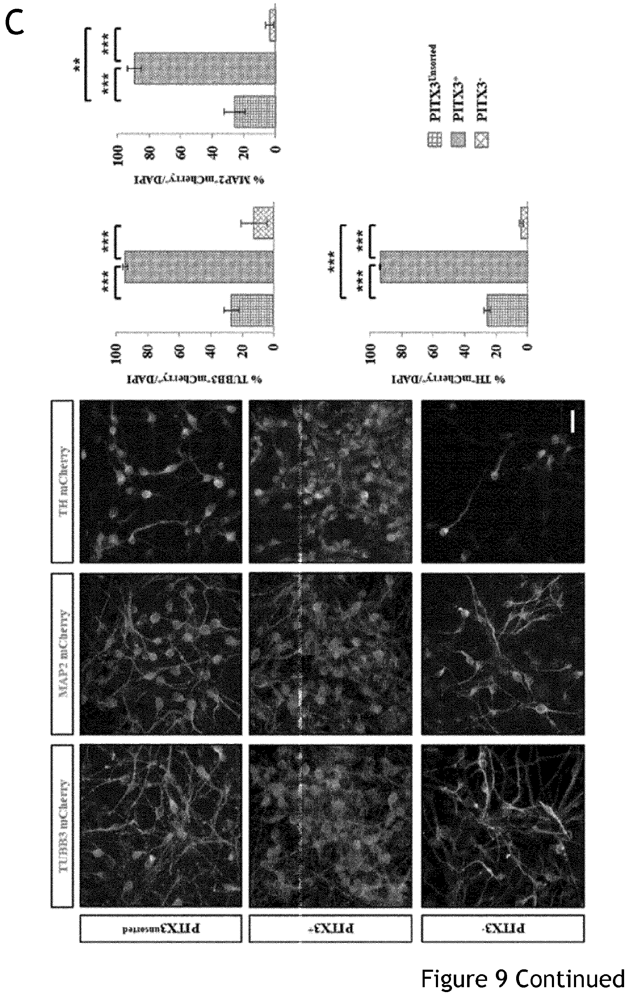

As can be seen in FIG. 5, mCherry expression was absent up until 30 days of differentiation (d30). mCherry-positive (mCherry⁺) neuron clusters were visualized on around d40. Meanwhile, the expression pattern of PITX3 gene was identical to that of its reporter mCherry throughout the differentiation (maturation) process. Terminally differentiated mDA neuron cultures were found to consist of ~16% mCherry⁺ cells coexpressing regional and lineage markers (EN1 and FOXA2, and LMX1A) (see FIG. 9).

Taken together, these data indicated that eGFP and mCherry expressions dependably recapitulated LMX1A and PITX3 expressions, respectively, at different time points during the mDA neuron differentiation.

Taken together, these data indicated that mCherry-expressing cells expressed PITX3 (construction of PITX3 reporter lines) and hESCs were directed to differentiate into mDA neuron with floor plate (FOXA2), midbrain (EN1), and mDA (LMX1A) characteristics.

### EXPERIMENTAL EXAMPLE 3: Characterization of LMX1A⁺ Cell

After exposure to 10 µM of Y27632 for 1 hr, the cells on d20 in Experimental Example 1 were dissociated with Accutase and strained through a 40 µm cell strainers (BD Science). The dissociated precursors were resuspended in LMX1A-Sorting Buffer (LMX1A-SB) supplemented with 3% fetal bovine serum (FBS) (Gemini Bio-Products), and 1x Penicillin-Streptomycin (P/S) (Gibco-Thermo Fisher Scientific) in HBSS (WELGENE, Inc., Gyeongsan, South Korea) at a final density of 2x10⁶ cells/ml. FACS was performed. Comparison of mRNA expression levels was made among the unsorted group, the LMX1A group, and the LMX1A⁺ group.

As can be seen in FIG. 6a, the LMX1A-eGFP⁺ (LMX1A⁺) fraction yielded ~41.1% of all viable cells after sorting. In addition, LMX1A⁺ and LMX1A-eGFP⁻(LMX1A⁻) progenitors appeared to retain similar morphologies to those of unsorted cells. Notably, ~99.4% of isolated LMX1A⁺ progenitor populations were positive for both EN1 and FOXA2. These results imply the separation of mDA neural precursor cells through FACS.

As can be seen in FIG. 6b, significant up-regulations of mDA progenitor-specific genes (EN1, FOXA1, FOXA2, LMX1A, and LMX1B) were observed in LMX1A⁺ group while serotonergic progenitor-specific gene (NKX2.2) and red nucleus (an anatomical area in the midbrain) progenitor-specific genes (SIM1, LHX1, and NKX6.1) were up-regulated in unsorted and LMX1A⁻ groups. These data suggest that LMX1A⁺ cells separated by FACS exhibit characteristics of mDA neural precursor cells.

Additionally, the unsorted group, the LMX1A⁻ group, and the LMX1A⁺ group were *in vitro* cultured for an additional one day after FACS. The cultured cells were observed for neuron-specific and proliferative cell-specific markers and subjected to BrdU assay to reveal various phases of the cell cycle.

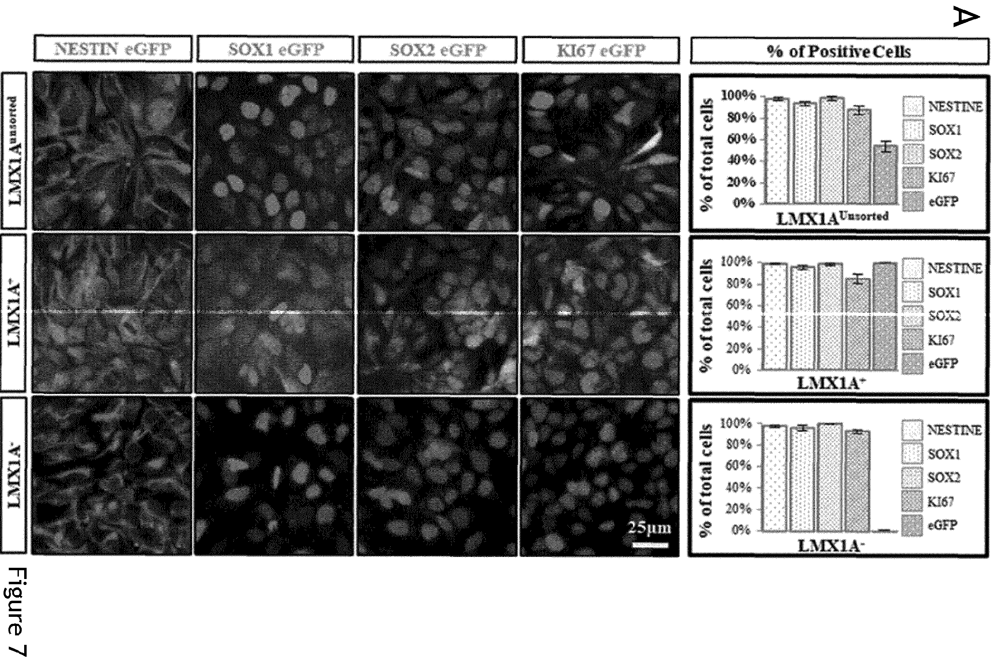

As can be seen in FIG. 7a, NESTIN-, SOX1-, SOX2- and KI67-positive cells were similar in all the three groups. These data suggest that the sorted LMX1A⁺ cells retain characteristics of neural progenitors and a proliferative potential, like the unsorted group and the LMX1A⁻ group.

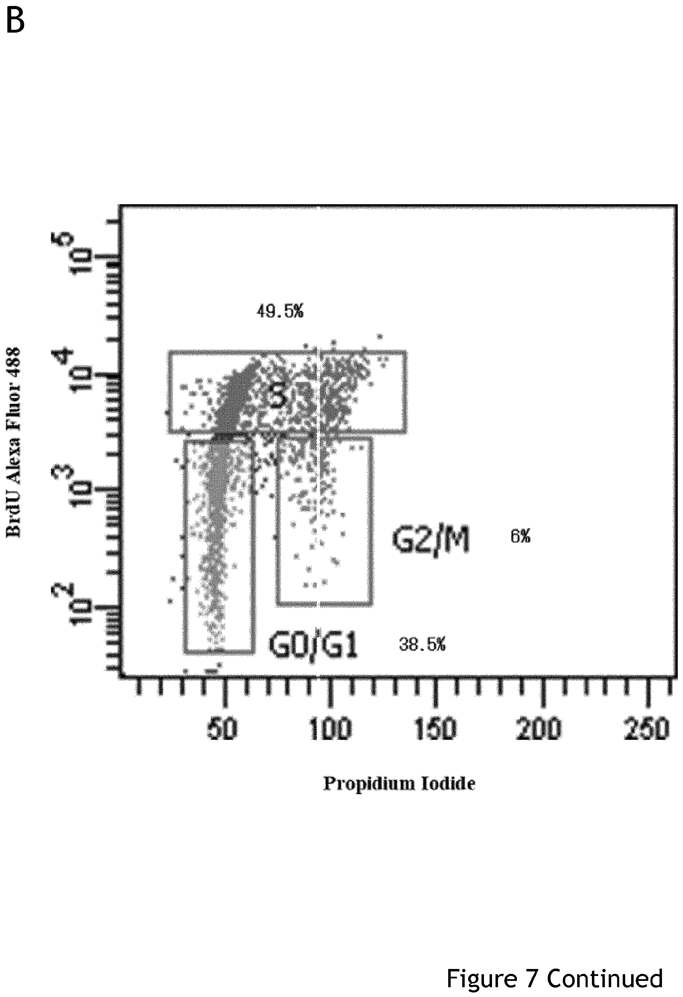

In LMX1A⁺ progenitor cultures, as shown in FIG. 7b, 38.5±3.9% and 49.5±6.2% of the viable cells at the mDA precursor stage were in the G0/G1 and S phases, respectively, while 6±2.7% were in the G2/M, indicating that the sorted LMX1A⁺ cells actually underwent the cell cycle for proliferation.

Finally, the unsorted group, the LMX1A⁻ group, and the LMX1A⁺ group after FACS were subjected to terminal differentiation (4 weeks, d52) and then compared with each other with respect to the expression of mDA neuron-specific markers.

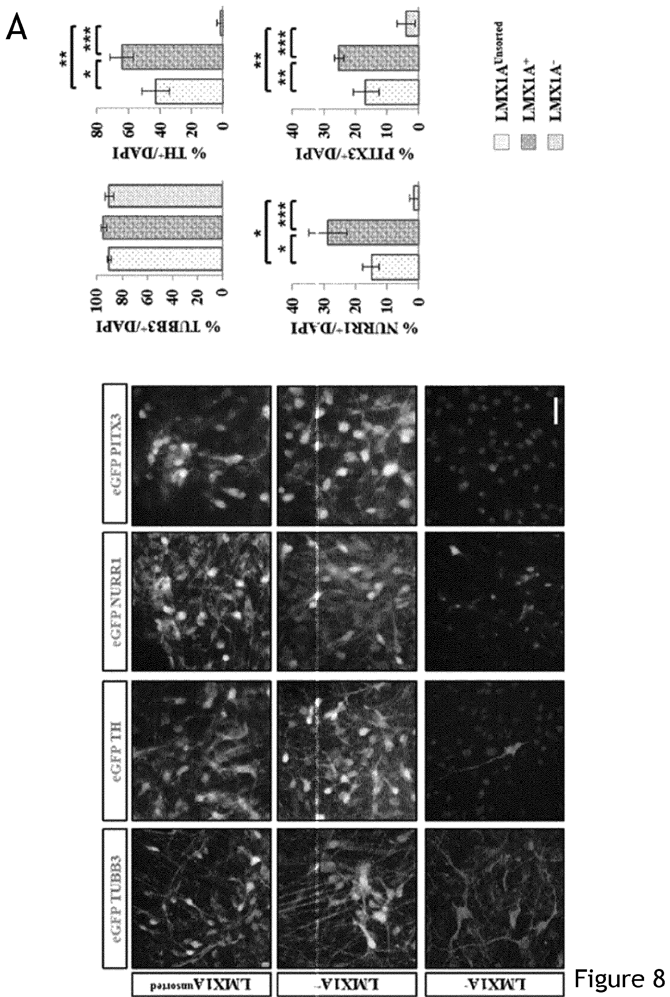

After the final differentiation, as can be seen in FIG. 8a, mDA neuron-specific genes (TH, NURR1, and PITX3) were significantly enriched in the LMX1A⁺ group, compared to the unsorted group and the LMX1A⁻ group, implying that the LMX1A⁺ cells are mDA precursors capable of differentiating to mDA neurons.

Further, the fully matured cells (8 weeks, d75) by the terminal differentiation were analyzed for dopamine release.

Briefly, cells were washed with a low KCl solution (2.5 mM CaCl₂, 11 mM glucose, 20 mM HEPES-NaOH, 4.7 mM KCl, 1.2 mM KH₂PO₄, 1.2 mM MgSO₄, and 140 mM NaCl) and incubated in the low KCl solution for 2min. The solution was subsequently replaced with a high KCl solution (2.5 mM CaCl₂, 11 mM glucose, 20 mM HEPES-NaOH, 60 mM KCl, 1.2 mM KH₂PO₄, 1.2 mM MgSO₄, and 85 mM NaCl), followed by incubation for an additional 15 min. The solution was collected in 15 ml tubes and centrifuged for 1 min at 2,000 rpm to remove the debris. The supernatant were collected in 1.5 ml tubes and stored at -80°C before the assay. The concentration of dopamine was detected by Dopamine ELISA kit (Cat. No. KA3838; Abnova, Taiwan) according to the manufacturer's instructions.

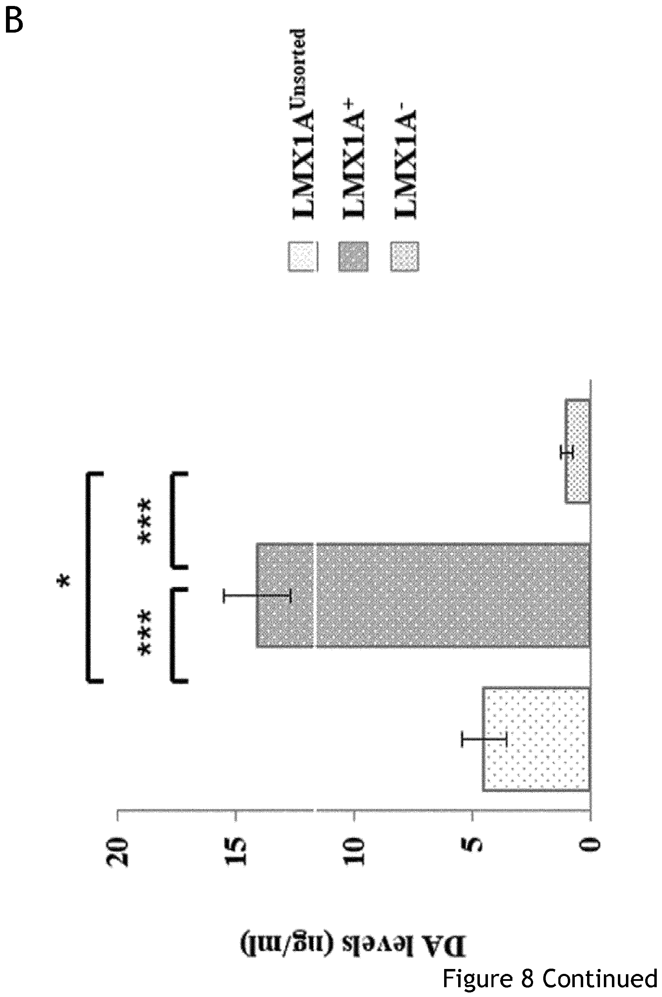

As can be seen in FIG. 8b, an increased release of dopamine was observed in the LMX1A⁺ group, compared to the unsorted group and the LMX1A⁻ group, after terminal differentiation. These data imply the mDA neurons to which the LMX1A⁺ cell have finally differentiated are functional neurons that release dopamine.

### EXPERIMENTAL EXAMPLE 4: Characterization of PITX3⁺ Cell

After being dissociated into single cells using Papain (Worthington Biochemical Corp.) supplemented with 5% trehalose, the cells on d40 of Experimental Example 2 were strained through 70 µm and 40 µm cell strainers, sequentially. The cells thus collected through the 40 µm cell strainer were resuspended in PITX3-Sorting Buffer (PITX3-SB) containing 5% FBS, 1 X Glutamax (Gibco-Thermo Fisher Scientific), 5% trehalose, and 1X P/S in HBSS at a concentration of 1x10⁷ cells/ml, followed by performing FACS. In addition, observation was made of morphologies of the cells that survived *in vitro* incubation for an additional 36 hrs after FACS.

As can be seen in FIG. 9a, the PITX3-mCherry⁺(PITX3⁺) fraction accounted for ~16% of all the viable cells after FACS. Although a significant cell loss was encountered during the cell sorting procedures, surviving PITX3⁺ and PITX3-mCherry⁻ (PITX3⁻) cells appeared to have similar neuronal morphologies to those of the unsorted cells after *in vitro* incubation. These results imply the separation of PITX3⁺ cells through FACS and the appearance of neuron-specific morphologies in all the three groups (unsorted, PITX3⁺, and PITX3⁻ groups).

Next, expression levels of mDA neuron-specific markers were compared among the unsorted group, the PITX3⁻ group, and the PITX3⁺ group of the cells on d40.

As can be seen in FIG. 9b, neuron-specific genes (NeuN and MAP2) and mDA neuron-specific genes (PITX3, NURR1, TH, DAT, and VMAT2) were significantly upregulated in the PITX3⁺ group, but with the expression of serotonergic neuron-specific gene (HTR2B) downregulated therein. In addition, as can be seen in FIG. 9c, neurons expressing the mDA neuron marker TH and neurons coexpressing the neuron-specific markers TUBB3 (TUJ1) and MAP2 were enriched in the PITX3⁺ group, suggesting that the PITX3⁺ cells are mDA neurons.

Finally, the post-FACS, unsorted group was additionally incubated and then subjected to double-labeled immunostaining on d44.

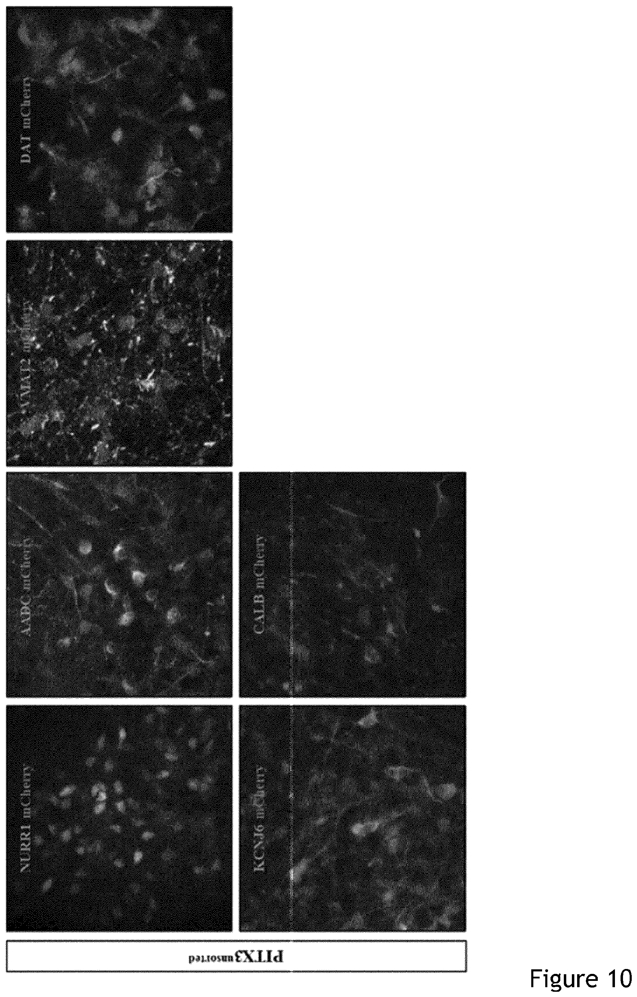

As can be seen in FIG. 10, PITX3⁺ cells were revealed to express NURR1, AADC, VMAT2, and DAT, which are all markers for mature mDA neurons. In addition, the A9 regional marker KCNJ6 (GIRK2) was expressed, but cells expressing the A10 regional marker CALB were not observed. These data suggest that the PITX3⁺ cells differentiated through the differentiation protocol of the present disclosure are mature mDA neural cells.

### EXPERIMENTAL EXAMPLE 5: Identification of Transplantation Suitability

After the LMX1A-eGFP reporter line of Preparation Example 1 and the PITX3-mCherry reporter line of Preparation Example 2 were differentiated using the differentiation protocol of the Example, mDA neural precursors on d20 and mature mDA neural cells on d50 were dissociated into single cells in the same manners as in Experimental Examples 3 and 4, respectively. Isolated single cells were compared for *in vitro* cell death. In this regard, cell death was measured using LIVE/DEAD^{™} Fixable Violet Dead Cell Stain kit (Thermo Fisher) according to the manufacturer's instruction.

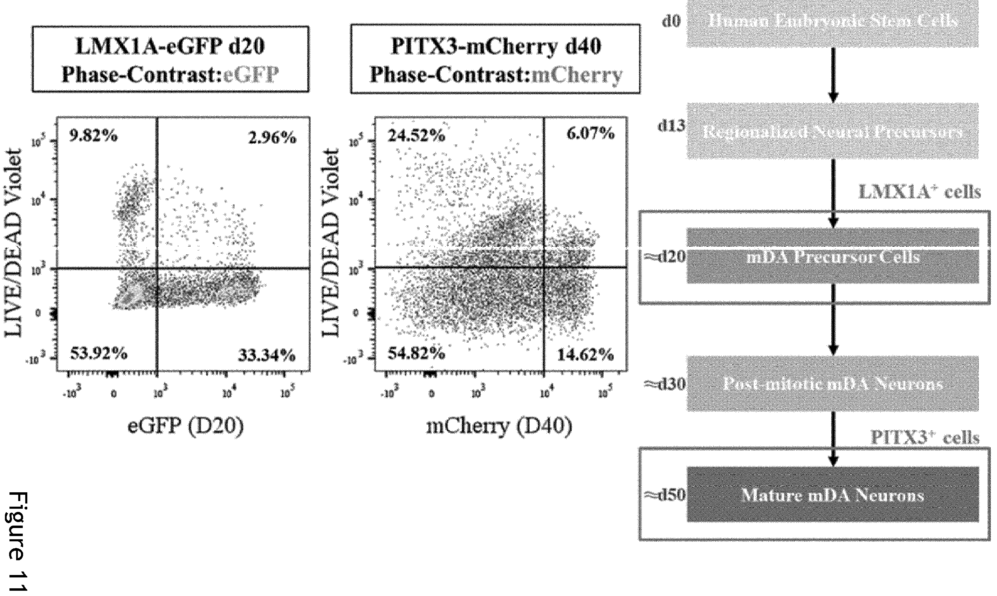

As can be seen in FIG. 11, the cells undergoing cell death after single cell dissociation accounted for about 8% of the LMX1A⁺ cells and about 30% of the PITX3⁺ cells. That is, when dissociated into single cells, LMX1A⁺ mDA neural precursors retained higher viability than PITX⁺ mDA neurons, demonstrating that there is a difference in susceptibility to single cell dissociation for transplantation between LMX1A⁺ cells and PITX3⁺ cells. These results imply that LMX1A⁺ cells, which are mDA neural precursors, are more advantageously transplanted than PITX3⁺ cells, which are mature neurons, in terms of cell death.

### EXPERIMENTAL EXAMPLE 6: Identification of mDA Marker

### 6-1. Transcriptome analysis

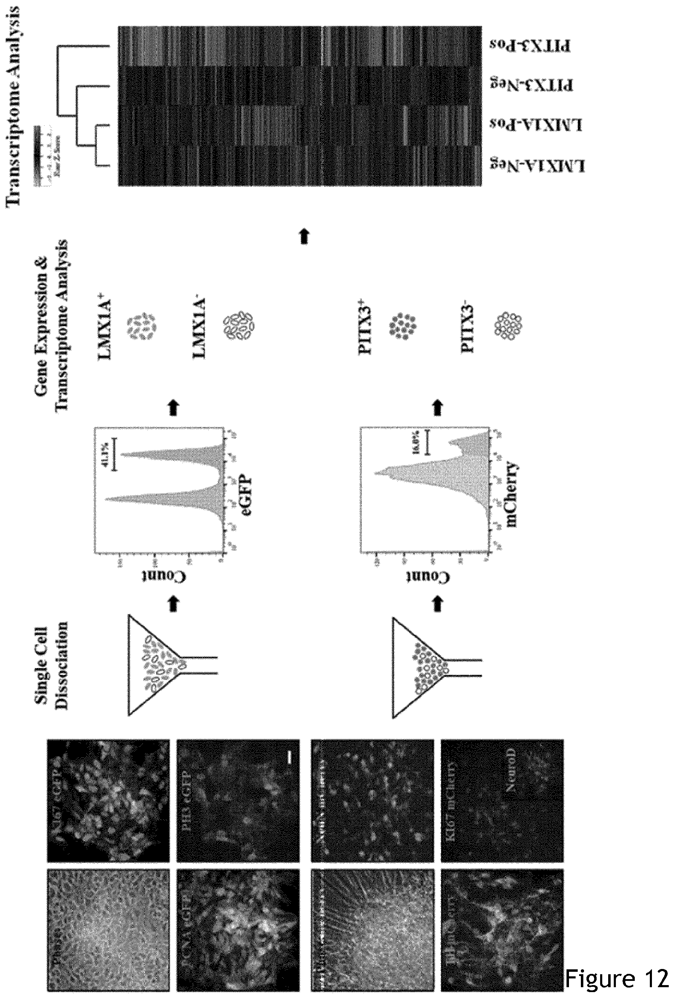

After the LMX1A-eGFP reporter line of Preparation Example 1 and the PITX3-mCherry reporter line of Preparation Example 2 were differentiated using the differentiation protocol of the Example, LMX1A⁺ and LMX1A⁻ cells on d20 (mDA neural precursor stage) and PITX3⁺ and PITX3⁻ cells on d40 (mDA neuron state) were separated and subjected to transcriptome analysis (Microarray) (see FIG. 12).

Meanwhile, coexpression of eGFP and cell cycle markers (Ki67, PCNA, and PH3) were detected in mDA progenitors and cells at the mDA neuron stage were observed to express mCherry and mDA neuronal marker (TH) and mature neuron marker (NeuN), but not to express immature neuron maker (NeuroD) and proliferative cell marker (Ki67).

### 6-2. Identification of mDA marker candidates

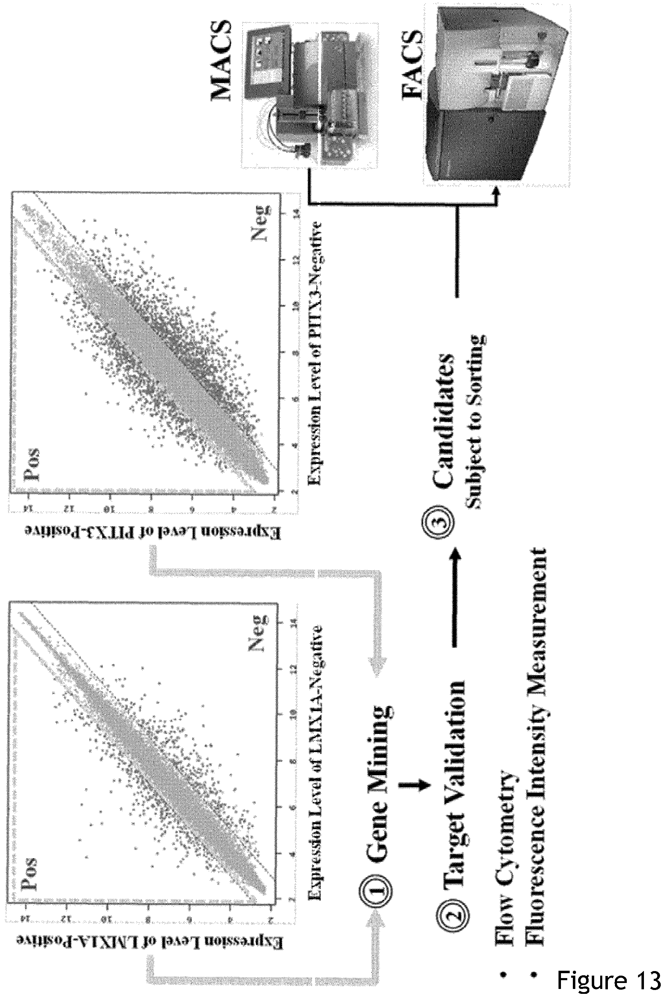

Based on the above result, mDA marker candidates were obtained. A concrete procedure is depicted in FIG. 13.

Comparative microarray analysis of the four isolated cells identified upregulated genes in LMX1A⁺ cells and PITX3⁺ cells relative to their reference cells LMX1A⁻ cells and PITX3⁻ cells (>2-FC). Among the upregulated genes, 53 candidate genes coding for cell membrane proteins having extracellular domains were identified by gene mining. The 53 identified genes included a number of genes known to be specific for mouse mDA progenitors *(Corin, Clstn2, Kitlg, Plxdc2, Pcdh7, Ferd31, Freml, Alcam,* and *Notch2*)*.*

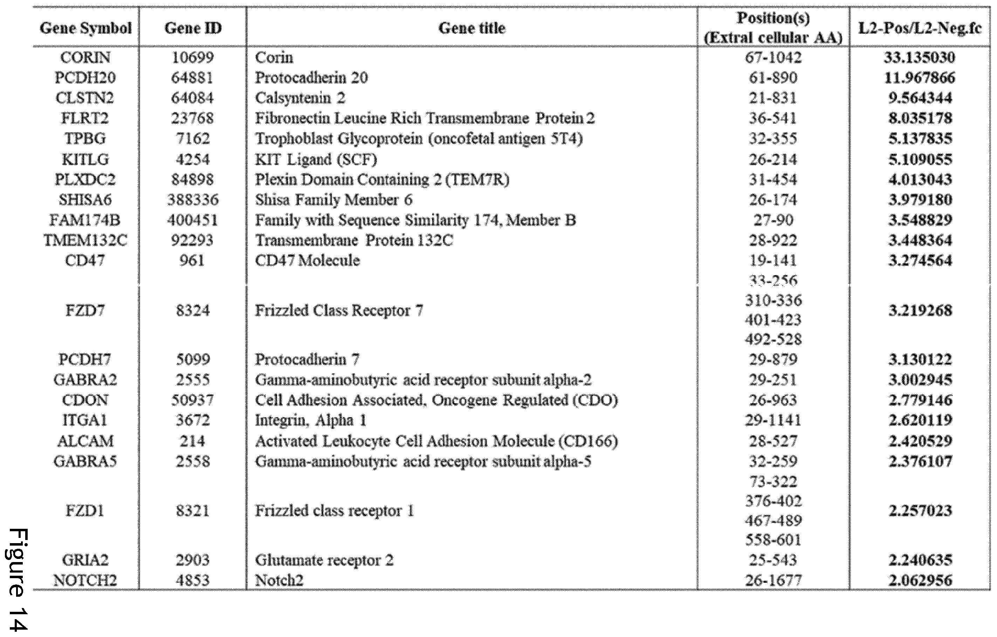

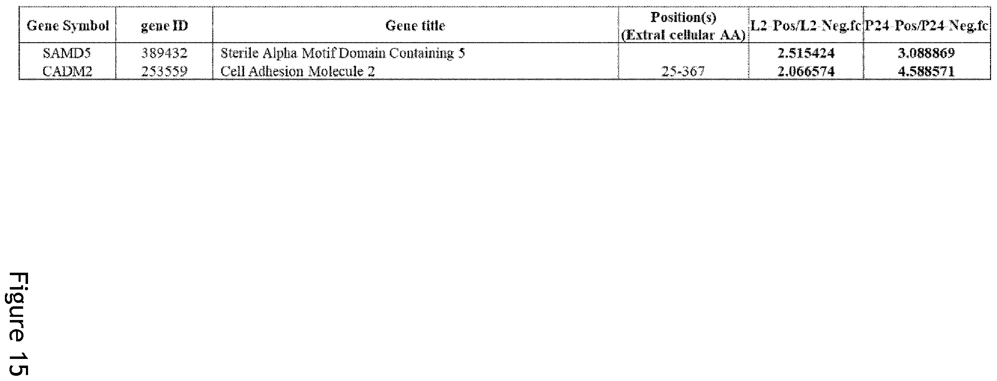

Subsequently, target validation was assessed by examining whether the genes were expressed in mDA cells that were practically differentiating. As a result, among the targets, surface marker genes were identified to be upregulated in LMX1A⁺ cells relative to LMX1A⁻ cells (FIG. 14) and upregulated or downregulated in both LMX1A⁺ cells and PITX3⁺ cells (FIG. 15). Commercially available antibodies were screened against 18 genes among 21 genes in FIG. 14.

Of the 18 genes, 4 *(CORIN, TPBG, CD47,* and *ALCAM*) were selected as final surface marker candidates.

### 6-3. Identification of mDA neural precursor-specific marker

Based on the above result, MACS targeting the four genes (*CORIN, TPBG, CD47,* and *ALCAM*) was performed.

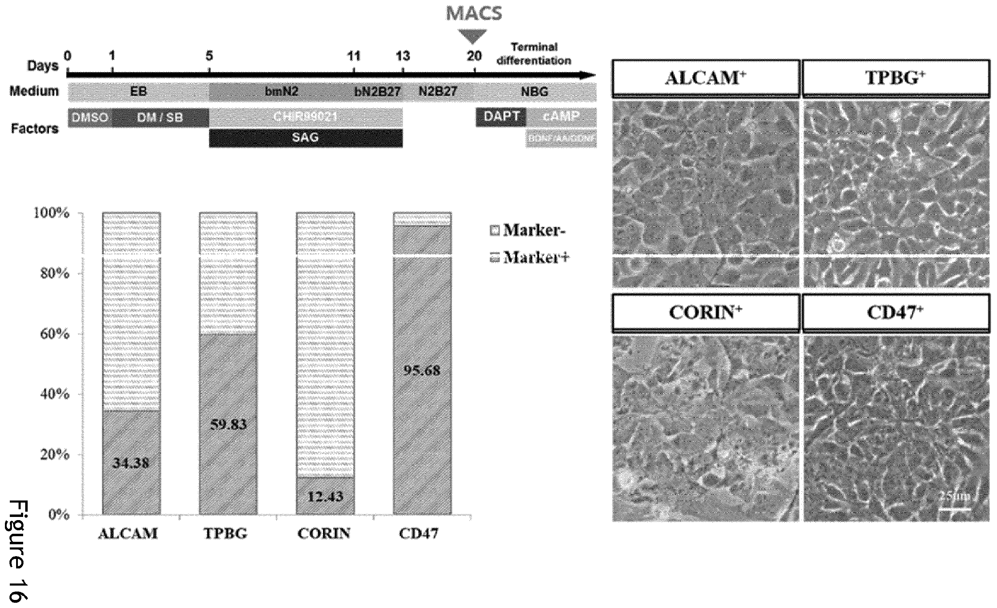

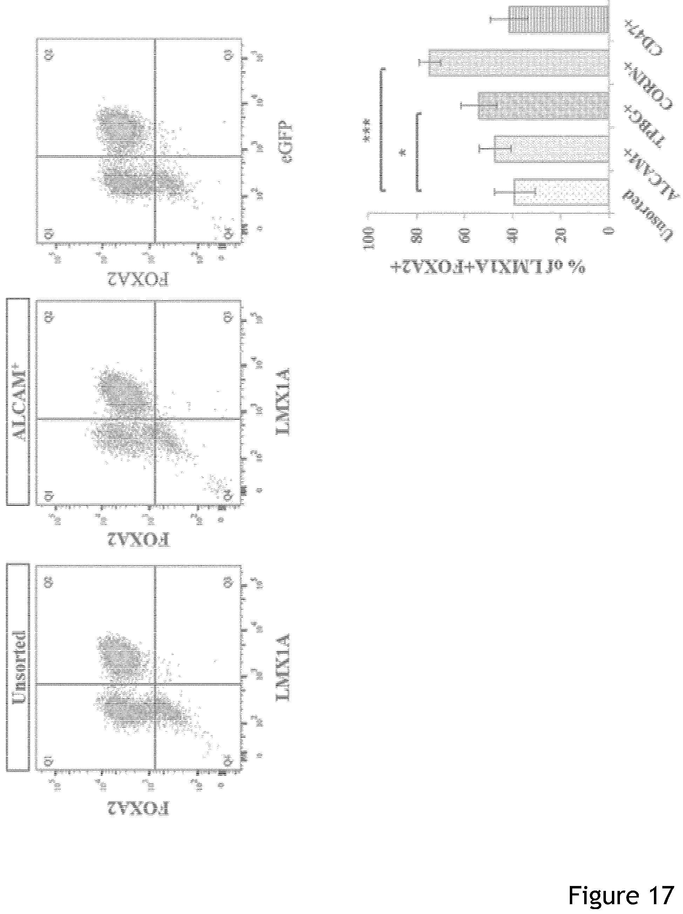

As can be seen in FIGS. 16 and 17, CORIN- and TPBG (trophoblast glycoprotein)-targeted MACS resulted in statistically significant enrichments of LMX1A⁺FOXA2⁺ mDA progenitors. Particularly, TPBG was expressed extensively in mDA progenitor culture population.

The CORIN gene was already known for use in enriching mDA progenitors whereas TPBG had never been previously reported in the context of mDA development. Finally, TPBG was thus selected as an mDA progenitor-specific marker.

### EXPERIMENTAL EXAMPLE 7: Effect of In vivo Transplantation of TPBG-Positive Cell Separated from Human Embryonic Stem Cell (hESC)

### 7-1. Construction of 6-OHDA-lesioned Parkinson's disease (PD) model

Female Sprague-Dawley rats weighing 200-250 g (Orient Bio Inc., Korea) were used as subjects to be transplanted. A combination of 30 mg/kg Zoletil^{®} (Virbac, France) and 10 mg/kg Rompun^{®} (Bayer, Germany) was used as an anesthesia.

According to coordinates (TB -0.45, AP -0.40, ML - 0.13, DV -0.70), 3 µL of 30 mM 6-OHDA was injected into the medial forebrain bundle of the rats to induce a hemi-parkinsonian model.

### 7-2. Behavioral recovery of PD-model after TPBG-positive cell transplantation

The hESC cultured in colonies were differentiated using the differentiation protocol of the Example and MACS targeting TPBG was performed after 20 days of differentiation (d20).

The dissociated TPBG-positive cells were suspended at a final concentration of 8.75×10⁴ cells/µL in 1X HBSS to give a cell suspension. For a control, HBSS alone was used.

Four weeks postlesion with 6-OHDA of Experimental Example 7-1, 4 µL of the cell suspension (total of 350,000 cells) was stereotaxically transplanted per rat at the coordinates (TB -0.24, AP +0.08, ML -0.30, DV - 0.40, and -0.50).

Immunosuppressive treatment was made for the duration of the experiment by intraperitoneally injecting cyclosporine A (Chong Kun Dang, Korea) every day at a dose of 10 mg/kg from 2 days prior to transplantation to the sacrifice of rats.

Amphetamine (2.5 mg/kg; Sigma-Aldrich) was intraperitoneally injected before transplantation, 4, 8, 12, or 16 weeks after transplantation and the amphetamine-induced rotation test was recorded for 30 minutes after injection.

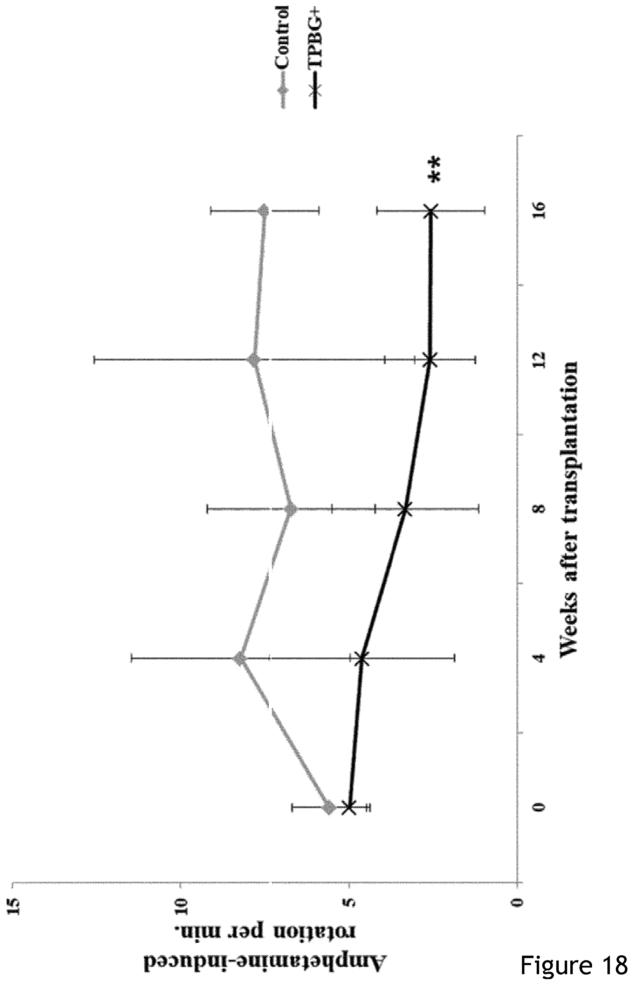

As can be seen in FIG. 18, the TPBG-positive cells exhibited a significantly improved motor function for 16 weeks post-transplantation, compared to the control. These data demonstrate that hESC-derived TPBG-positive mDA neural precursor cells are viable *in vivo* and improve a motor function.

### 7-3. Characterization of graft after TPBG-positive cell transplantation

TPBG-positive cells and unsorted cells were transplanted in the same manner as Example 7-2, with the exception that unsorted cells were used for a control

Sixteen weeks post-transplantation, the rats were anesthetized with 25% urethane solution and transcardially perfused with 0.9% saline solution followed by 4% paraformaldehyde. Removed brains were fixed overnight and cryoprotected in 30% sucrose-PBS solution. Cryoprotected brains were embedded in FSC 22^{®} compound (Leica, Nußloch, Germany), and coronal sections, each 18 µm thick, were made using a cryostat (Thermo Fisher Scientific). Then, immunohistochemistry against hNCAM (human-specific neural cell adhesion molecule) was carried out.

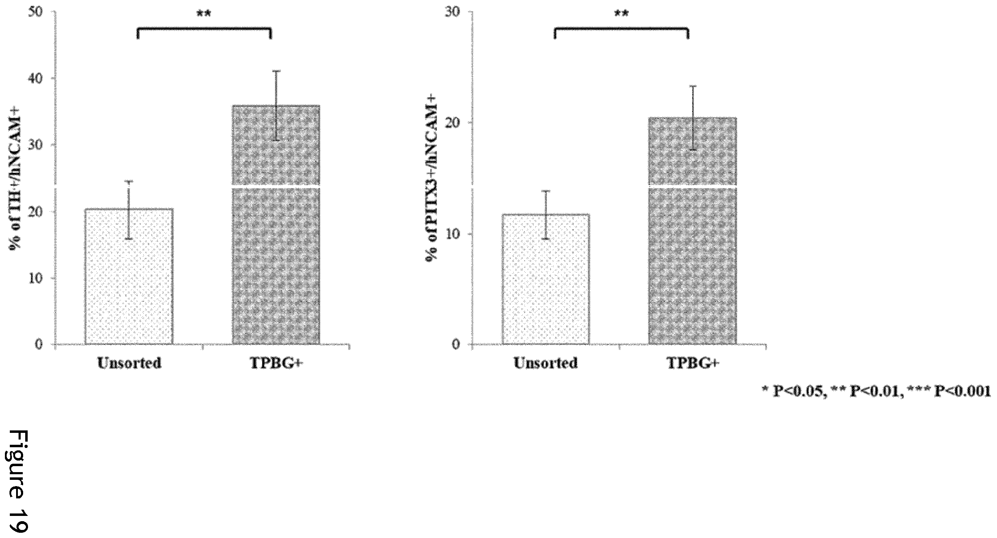

As can be seen in FIG. 19, the TPBG-positive cell group was composed of a greater number of TH⁺hNCAM⁺ and PITX3⁺hNCAM⁺ mDA neural cells, compared to the unsorted group. The result implies that TPBG-positive cells are more suitable for *in vivo* differentiation into mDA neurons, compared to the unsorted group.

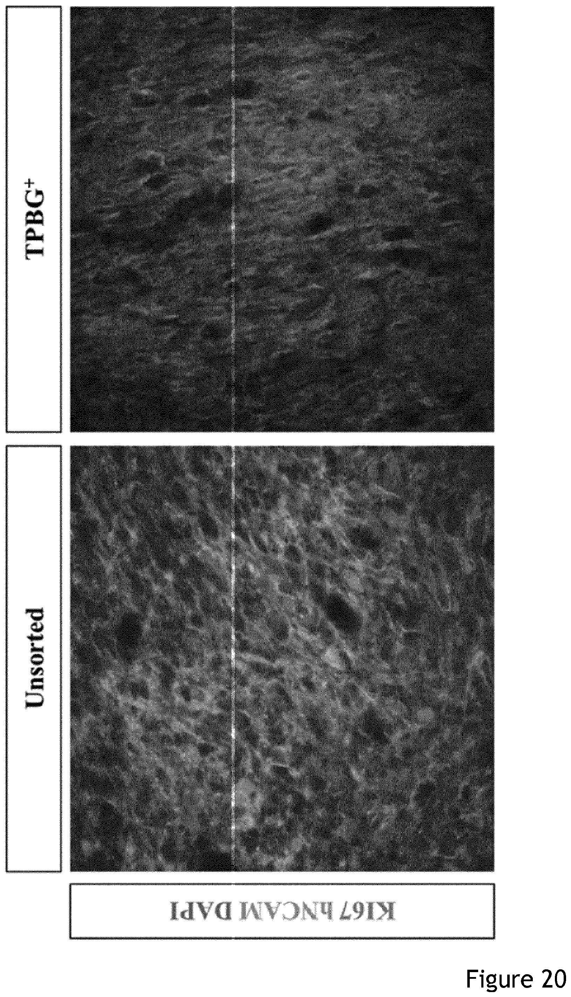

In addition, as can be seen in FIG. 20, a graft comprising about 20 % or more of KI67⁺hNCAM⁺ cells was observed in one certain rat in the unsorted group while no KI67⁺hNCAM⁺ cells were found in the TPBG-positive group. These data indicate that the unsorted group has the feasibility of retaining proliferative potential even after 16 weeks post-transplantation, but proliferative cells can be excluded upon cell sorting by TPBG. The result is very significant in terms of safety for cell therapy.

### EXPERIMENTAL EXAMPLE 8: Characterization of TPBG-Positive Cell Separated from Human Fetal Ventral Mesencephalic Cells (fVM Cells)

When cultured on a laminin-coated plate in fVM cells in a nerve stem cell maintenance medium (ReNcell NSC maintenance Medium, Merck) at confluence 80-90 %, fVM cells were subjected to TPBG-targeting MACS. Then, the expression of EN1 in TPBG-positive cells and TPBG-negative cells was analyzed by qRT-PCR relative to the control hESC (H9) (expression of H9=1).

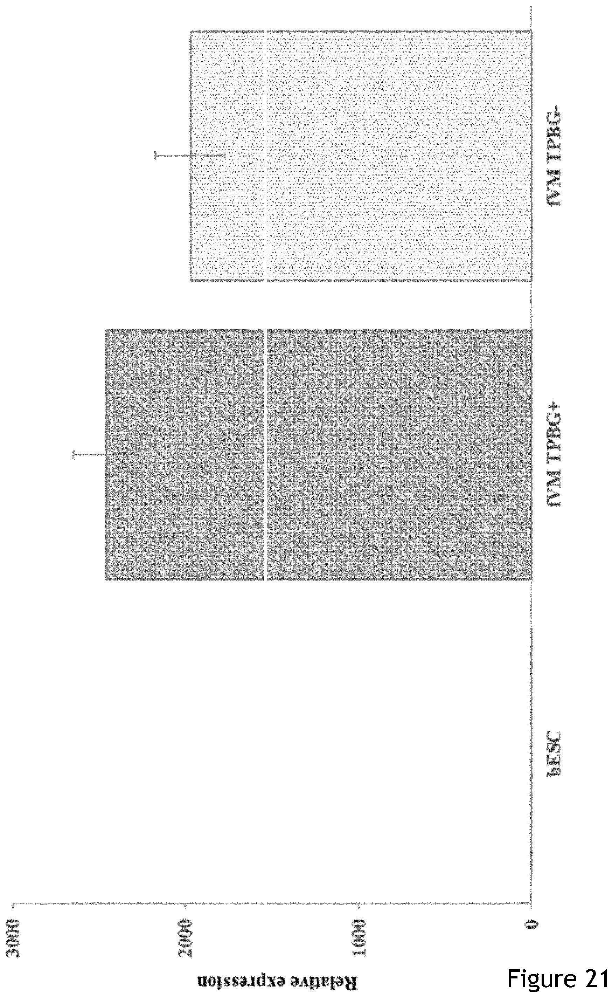

As can be seen in FIG. 21, the expression of the mDA neuron-specific regional marker EN1 in TPBG-positive cells was increased compared to TPBG-negative cells, indicating that TPBG can be used for enrichment of fVM cells exhibiting midbrain characteristics.

### EXPERIMENTAL EXAMPLE 9: Characterization of Human Induced Pluripotent Stem Cells (iPSC) Separated from TPBG-Positive Cell

Human iPSC (HDF-epi3) that was being cultured in the same manner as for the human embryonic stem cells was allowed to differentiate using the differentiation protocol of the Example and then subjected to MACS targeting TPBG on d20. The separated TPBG-positive cells were assayed for expression of EN1, FOXA2, and LMX1A (Immunocytochemistry).

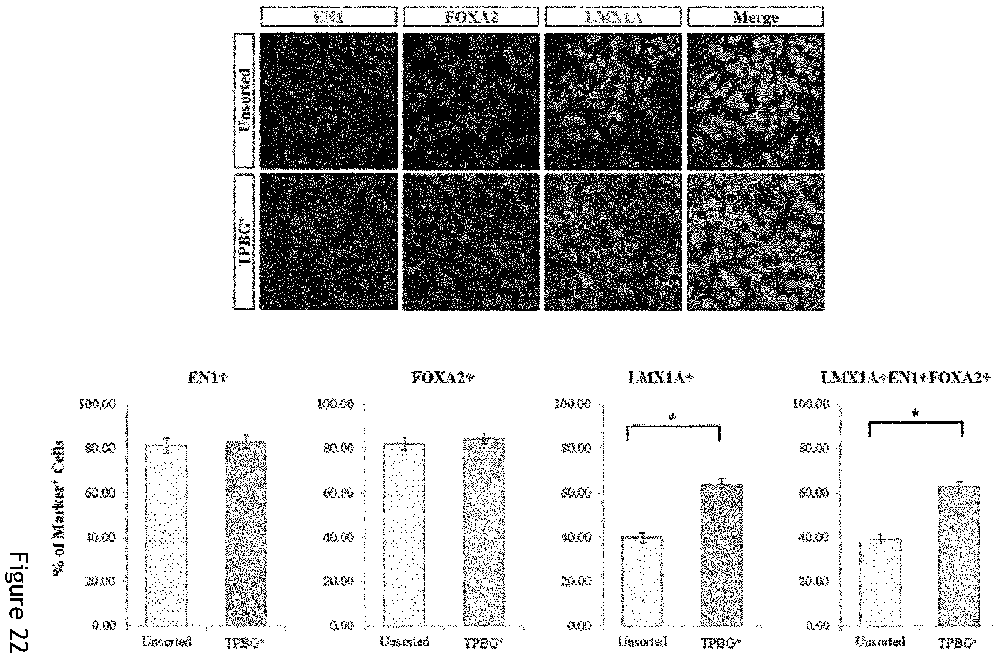

As can be seen in FIG. 22, the expression of the midbrain-specific regional markers EN1 and FOXA2 did not differ before and after MACS, but TPBG-positive cells expressing the mDA lineage marker LMX1A were enriched. In addition, TPBG-positive cells positive for all the three markers (EN1, FOXA2, and LMX1A) were also significantly enriched.

## Claims

1. A method for preparation of dopaminergic neural cells, the method comprising:
(a) contacting a cell population comprising dopaminergic neural progenitors or dopaminergic neural precursor cells with TPBG (trophoblast glycoprotein) antibodies; and
(b) separating TPBG-positive dopaminergic neural cells, which bind to the TPBG antibodies.

2. The method of claim 1, wherein the dopaminergic neural progenitors are derived from the human stem cells or progenitors.

3. The method of claim 2, wherein the human stem cells or progenitors are embryonic stem cells, embryonic germ cells, embryonic carcinoma cells, induced pluripotent stem cells (iPSCs), adult stem cells, or fetal cells.

4. The method of claim 3, wherein the fetal cells are derived from a fetal neural tissue or derivatives thereof.

5. The method of claim 1, wherein the TPBG-positive dopaminergic neural cells alleviate symptoms of Parkinson's disease.

6. The method of claim 1, wherein the TPBG-positive dopaminergic neural cells improve safety for transplantation.

7. The method of claim 1, wherein the dopaminergic neural cells are midbrain dopaminergic neural cells, which are optionally A9 region-specific midbrain dopaminergic neural cells.

8. A pharmaceutical composition comprising TPBG (trophoblast glycoprotein)-positive dopaminergic neural cells for use in treatment of Parkinson's disease, wherein the TPBG-positive dopaminergic neural cells are generated according to the method of claim 1.

9. The pharmaceutical composition for use of claim 8, wherein the dopaminergic neural cells are midbrain dopaminergic neural cells, which are optionally A9 region-specific midbrain dopaminergic neural cells.

10. An in vitro method for enhancing the efficacy and improving transplantation safety of dopaminergic neural cells in transplantation for Parkinson's disease, the method comprising:
(a) contacting a cell population comprising dopaminergic neural progenitors or dopaminergic neural precursor cells with TPBG (trophoblast glycoprotein) antibodies; and
(b) separating TPBG-positive dopaminergic neural cells, which bind to the TPBG antibodies.

11. The method of claim 10, wherein the dopaminergic neural progenitors are derived from the human stem cells or progenitors.

12. The method of claim 11, wherein the human stem cells or progenitors are embryonic stem cells, embryonic germ cells, embryonic carcinoma cells, induced pluripotent stem cells (iPSCs), adult stem cells, or fetal cells, wherein optionally the fetal cells are derived from a fetal neural tissue or derivatives thereof.

13. The method of claim 10, wherein the dopaminergic neural cells are midbrain dopaminergic neural cells, which are optionally A9 region-specific midbrain dopaminergic neural cells.

## Patentansprüche

1. Verfahren zur Herstellung dopaminerger Nervenzellen, das Verfahren umfassend:
(a) Inkontaktbringen einer Zellpopulation, umfassend dopaminerge neuronale Vorläuferzellen, mit TPBG-Antikörpern (Trophoblast-Glykoprotein); und
(b) Trennen von TPBG-positiven dopaminergen Nervenzellen, die an die TPBG-Antikörper binden.

2. Verfahren nach Anspruch 1, wobei die dopaminergen neuronalen Vorläuferzellen abgeleitet sind aus den menschlichen Stammzellen oder Vorläuferzellen.

3. Verfahren nach Anspruch 2, wobei die menschlichen Stammzellen oder Vorläuferzellen embryonale Stammzellen, embryonale Keimzellen, embryonale Karzinomzellen, induzierte pluripotente Stammzellen (iPS-Zellen), adulte Stammzellen oder fetale Zellen sind.

4. Verfahren nach Anspruch 3, wobei die fetalen Zellen abgeleitet sind aus einem fetalen Neuralgewebe oder Derivaten davon.

5. Verfahren nach Anspruch 1, wobei die TPBG-positiven dopaminergen Nervenzellen Symptome einer Parkinson-Krankheit lindern.

6. Verfahren nach Anspruch 1, wobei die TPBG-positiven dopaminergen Nervenzellen die Sicherheit für eine Transplantation verbessern.

7. Verfahren nach Anspruch 1, wobei die dopaminergen Nervenzellen dopaminerge Nervenzellen im Mittelhirn sind, die optional für die A9-Region spezifische dopaminerge Nervenzellen des Mittelhirns sind.

8. Pharmazeutische Zusammensetzung, umfassend TPBG (Trophoblast-Glykoprotein)-positive dopaminerge Nervenzellen für die Verwendung bei der Behandlung einer Parkinson-Krankheit, wobei die TPBG-positiven dopaminergen Nervenzellen gemäß dem Verfahren nach Anspruch 1 erzeugt werden.

9. Pharmazeutische Zusammensetzung für die Verwendung nach Anspruch 8, wobei die dopaminergen Nervenzellen dopaminerge Nervenzellen im Mittelhirn sind, die optional für die A9-Region spezifische dopaminerge Nervenzellen des Mittelhirns sind.

10. In-vitro-Verfahren zum Steigern der Wirksamkeit und Verbessern der Transplantationssicherheit dopaminerger Nervenzellen bei der Transplantation für die Parkinson-Krankheit,
das Verfahren umfassend:
(a) Inkontaktbringen einer Zellpopulation, umfassend dopaminerge neuronale Vorläuferzellen, mit TPBG-Antikörpern (Trophoblast-Glykoprotein); und
(b) Trennen von TPBG-positiven dopaminergen Nervenzellen, die an die TPBG-Antikörper binden.

11. Verfahren nach Anspruch 10, wobei die dopaminergen neuronalen Vorläuferzellen abgeleitet sind aus den menschlichen Stammzellen oder Vorläuferzellen.

12. Verfahren nach Anspruch 11, wobei die menschlichen Stammzellen oder Vorläuferzellen embryonale Stammzellen, embryonale Keimzellen, embryonale Karzinomzellen, induzierte pluripotente Stammzellen (iPS-Zellen), adulte Stammzellen oder fetale Zellen sind, wobei optional die fetalen Zellen abgeleitet sind aus einem fetalen Neuralgewebe oder Derivaten davon.

13. Verfahren nach Anspruch 10, wobei die dopaminergen Nervenzellen dopaminerge Nervenzellen im Mittelhirn sind, die optional für die A9-Region spezifische dopaminerge Nervenzellen des Mittelhirns sind.

## Revendications

1. Procédé de préparation de cellules neuronales dopaminergiques, le procédé comprenant :
(a) la mise en contact d'une population cellulaire comprenant des progéniteurs neuronaux dopaminergiques ou des cellules précurseurs neuronales dopaminergiques avec des anticorps TPBG (glycoprotéine trophoblastique), et
(b) la séparation des cellules neuronales dopaminergiques TPBG-positives, qui se lient aux anticorps TPBG.

2. Procédé selon la revendication 1, dans lequel les progéniteurs neuronaux dopaminergiques sont dérivés des cellules souches humaines ou de progéniteurs.

3. Procédé selon la revendication 2, dans lequel les cellules souches humaines ou les progéniteurs sont des cellules souches embryonnaires, des cellules germinales embryonnaires, des cellules de carcinome embryonnaires, des cellules souches pluripotentes induites (iPSC, induced pluripotent stem cells), des cellules souches adultes ou des cellules fœtales.

4. Procédé selon la revendication 3, dans lequel les cellules fœtales sont dérivées d'un tissu neuronal fœtal ou de dérivés de celui-ci.

5. Procédé selon la revendication 1, dans lequel les cellules neuronales dopaminergiques TPBG-positives atténuent les symptômes de la maladie de Parkinson.

6. Procédé selon la revendication 1, dans lequel les cellules neuronales dopaminergiques TPBG-positives améliorent la sécurité de la transplantation.

7. Procédé selon la revendication 1, dans lequel les cellules neuronales dopaminergiques sont des cellules neuronales dopaminergiques du mésencéphale, consistant éventuellement en des cellules neuronales dopaminergiques spécifiques à la région A9 du mésencéphale.

8. Composition pharmaceutique comprenant des cellules neuronales dopaminergiques TPBG (glycoprotéine trophoblastique)-positives destinée à une utilisation dans le traitement de la maladie de Parkinson, lesdites cellules neuronales dopaminergiques TPBG-positives étant générées selon le procédé de la revendication 1.

9. Composition pharmaceutique destinée à une utilisation selon la revendication 8, lesdites cellules neuronales dopaminergiques étant des cellules neuronales dopaminergiques du mésencéphale, consistant éventuellement en des cellules neuronales dopaminergiques spécifiques à la région A9 du mésencéphale.

10. Procédé *in vitro* pour améliorer l'efficacité et la sécurité de la transplantation des cellules neuronales dopaminergiques lors d'une transplantation relative à la maladie de Parkinson, le procédé comprenant :
(a) la mise en contact d'une population cellulaire comprenant des progéniteurs neuronaux dopaminergiques ou des cellules précurseurs neuronales dopaminergiques avec des anticorps TPBG (glycoprotéine trophoblastique), et
(b) la séparation des cellules neuronales dopaminergiques TPBG-positives qui se lient aux anticorps TPBG.

11. Procédé selon la revendication 10, dans lequel les progéniteurs neuronaux dopaminergiques sont dérivés de cellules souches humaines ou de progéniteurs.

12. Procédé selon la revendication 11, dans lequel les cellules souches humaines ou les progéniteurs sont des cellules souches embryonnaires, des cellules germinales embryonnaires, des cellules de carcinome embryonnaires, des cellules souches pluripotentes induites (iPSC, induced pluripotent stem cells), des cellules souches adultes ou des cellules fœtales, lesdites cellules fœtales étant éventuellement dérivées d'un tissu neuronal fœtal ou de dérivés de celui-ci.

13. Procédé selon la revendication 10, dans lequel les cellules neuronales dopaminergiques sont des cellules neuronales dopaminergiques du mésencéphale, consistant éventuellement en des cellules neuronales dopaminergiques spécifiques à la région A9 du mésencéphale.
